# EUROPEAN PATENT SPECIFICATION

(11) **EP 2 398 407 B1**
(45) Date of publication and mention of the grant of the patent: **22.03.2017**
(21) Application number: 09801611.6
(22) Date of filing: 16.12.2009
(51) Int. Cl.: A61B 17/32, A61B 17/34, A61B 17/00, A61B 17/3207

(54) **TISSUE REMOVAL DEVICE FOR NEUROSURGICAL SURGERY APPLICATIONS**
VORRICHTUNG ZUR ENTFERNUNG VON GEWEBE FÜR ANWENDUNGEN IN DER NEUROCHIRURGIE
DISPOSITIF D'ABLATION TISSULAIRE DESTINÉ À DES APPLICATIONS NEUROCHIRURGICALES

(30) Priority: 09.06.2009 US 481219; 24.02.2009 US 391579; 20.02.2009 US 389447; 16.03.2009 US 404407
(43) Date of publication of application: 28.12.2011
(73) Proprietor: Nico Corporation, Indianapolis, IN 46240 (US)
(72) Inventor: MARK, Joseph, L., Indianapolis IN 46208 (US); DOUGHERTY, Brian, Terre Haute IN 47803 (US)
(74) Representative: McCartney, Jonathan William
(86) International application number: PCT/US2009/068329
(87) International publication number: WO 2010/096139

(56) References cited:
- EP-A1- 1 201 210
- EP-A2- 0 125 070
- WO-A1-02/30303
- WO-A1-94/18894
- WO-A1-96/13845
- WO-A1-2006/123312
- WO-A1-2007/002230
- WO-A2-2007/005507
- WO-A2-2007/047380
- US-A- 4 210 146
- US-A- 5 085 658
- US-A- 5 195 541
- US-A- 5 411 513
- US-A- 5 782 849
- US-A- 6 032 673
- US-A1- 2001 037 114
- US-A1- 2002 103 496
- US-A1- 2003 073 980
- US-A1- 2004 049 217
- US-A1- 2005 027 210
- US-A1- 2005 277 970
- US-A1- 2008 114 387
- US-A1- 2008 249 553
- US-A1- 2008 262 476
- US-B1- 6 402 701

## Description

### TECHNICAL FIELD

The present disclosure relates to tissue cutting devices, in particular, tissue cutting devices that are suited for neurosurgical surgical procedures.

### BACKGROUND

Various abnormalities of the neurological system, such as brain and spinal tumors, cysts, lesions, or neural hematomas, can cause severe health risks to patients afflicted by them, including deterioration in motor skills, nausea or vomiting, memory or communication problems, behavioral changes, headaches, or seizures. In certain cases, resection of abnormal tissue masses is required. However, given the complexity and importance of the neurological system, such neurosurgical procedures are extremely delicate and must be executed with great precision and care. Many known tissue cutting devices suffer from an inability to quickly and cleanly sever neurological tissue samples without causing "traction" or pull on the surrounding tissue. In addition, many known devices are not configured to both "debulk" large structures and to finely shave smaller, more delicate structures and lack the flexibility needed in many procedures. Furthermore, many neurological procedures impose significant space limitations on the surgeon, and the tissue resection device needs to be manipulable by the surgeon with one hand in relatively small spaces, such prior art devices are e.g. disclosed by US 2005/0027210 A1, US 5,782,498 A1, us2004/0049217 A1, US 4,032,673 A1 or US 6,402,701 B1, wherein US 2005/0027210 A1 is considered as closest prior art for the subject-matter of claim 1. Many known devices, such as e.g. US 6,032,673 A1, either emulsify the resected tissue, macerate the resected tissue, or thermally damage the tissue rendering it unsuitable for subsequent analysis (e.g., pathologic and/or histologic analysis) which is necessary for the determination of the most effective post resection treatment therapies. Thus, a need has arisen for a tissue cutting device that addresses the foregoing issues.

The problems are solved by the invention as disclosed in independent claim 1 with preferred embodiments disclosed in the dependent claims.

### BRIEF DESCRIPTION OF THE DRAWINGS

Embodiments of the present disclosure will now be described by way of example in greater detail with reference to the attached figures, in which:
FIG. 1 is a perspective view of a tissue cutting device in accordance with a first embodiment;
FIG. 2 is a cross-sectional view of the tissue cutting device of FIG. 1 depicting an inner cannula in a first relative position with respect to an outer cannula in which the inner cannula's distal end is located proximally of the outer cannula's distal end;
FIG. 3 is a cross-sectional view of the tissue cutting device of FIG. 1 depicting the inner cannula in a second relative position with respect to the outer cannula in which the inner cannula's distal end is located at the distal end of the outer cannula;
FIG. 4 is a partial cross-sectional view of the tissue cutting device of FIG. 1 in a first configuration in which a device-mounted tissue collector is disconnected from a tissue cutting device housing;
FIG. 5 is a partial cross-sectional view of the tissue cutting device of FIG. 4 in a second configuration in which the device-mounted tissue collector is connected to the tissue cutting device housing;
FIG. 6 is a partial cross-sectional view of an alternate embodiment of the tissue cutting device of FIG. 1 in a first configuration in which the device-mounted tissue collector is disconnected from the tissue cutting device;
FIG. 7 is partial cross-sectional view of the tissue cutting device of FIG. 6 in a second configuration in which the device-mounted tissue collector is connected to the tissue cutting device;
FIG. 8 is a broken side elevation view of the outer cannula of the tissue cutting device of FIG. 1;
FIG. 9 is a broken side elevation view of the inner cannula of the tissue cutting device of FIG. 1;
FIG. 10 is a top plan view of a portion of the outer cannula of the tissue cutting device of FIG. 1 with the inner cannula removed from the outer cannula;
FIG. 11 is a top plan view of a portion of the inner cannula of the tissue cutting device of FIG. 1;
FIG. 12 is a top plan view of a portion of the outer cannula and inner cannula of FIG. 1 depicting the inner cannula inserted into the outer cannula;
FIG. 13 is a partial cross-sectional view of a distal region of the outer cannula and the inner cannula of the tissue cutting device of FIG. 1, depicting the inner cannula in a first relative position with respect to the outer cannula;
FIG. 14 is a partial cross-sectional view of a distal region of the outer cannula and the inner cannula of the tissue cutting device of FIG. 1, depicting the inner cannula in a second relative position with respect to the outer cannula;
FIG. 15 is an exploded assembly view of the tissue cutting device of FIG. 1;
FIG. 16a is a side elevation view of a cam of the tissue cutting device of FIG. 1;
FIG. 16b is an end elevation view of the cam of FIG. 16a;
FIG. 17a is a perspective view of a cam transfer mechanism of the tissue cutting device of FIG. 1;
FIG. 17b is a perspective view of a cam follower of the tissue cutting device of FIG. 1;
FIG. 18 is a partial perspective view of a portion of the tissue cutting device of FIG. 1 with an upper shell of an outer sleeve upper housing removed to show a dial for rotating the outer cannula;
FIG. 19 is a partial side cross-sectional view of the portion of the tissue cutting device of FIG. 18;
FIG. 20 is a side elevation view of an inner and outer cannula assembly of the tissue cutting device of FIG. 1;
FIG. 21A is a tissue cutting system including a remote tissue collector, control console, foot pedal, and the tissue cutting device of FIG. 1;
FIG. 21B is an enlarged view of the remote tissue collector of FIG. 21A;
FIG. 22 is a block diagram of a control scheme for the tissue cutting system of FIG. 21A;
FIG. 23 is diagram of the tissue cutting device of FIG. 1 and the motor control unit of FIG. 22;
FIG. 24 is a partial cross-sectional view of the tissue cutting device of FIG. 1 depicting motor shaft position sensors for controlling a stop position of an inner cannula;
FIG. 25 is a partial cross-sectional view of the outer cannula and inner cannula of the tissue cutting device of FIG. 1 with the inner cannula in a first position relative to the outer cannula;
FIG. 26 is a partial cross-sectional view of the outer cannula and inner cannula of the tissue cutting device of FIG. 1 with the inner cannula in a second position relative to the outer cannula;
FIG. 27 is a partial cross-sectional view of the outer cannula and the inner cannula of the tissue cutting device of FIG. 1 with the inner cannula in a third position relative to the outer cannula;
FIG. 28 is a rear elevational view of the tissue cutting device of FIG. 1;
FIG. 29A is a perspective view of a single hand gripping the tissue cutting device of FIG. 1 in a first gripping position;
FIG. 29B is a perspective view of a single hand gripping the tissue cutting device of FIG. 1 in a second gripping position;
FIG. 29C is a perspective view of a single hand gripping the tissue cutting device of FIG. 1 in a third gripping position
FIG. 30 is a side elevational view of an embodiment of an endoscope for use with the tissue cutting device of FIG. 1;
FIG. 31A is a side elevational view of an embodiment of a trocar for use with the endoscope of FIG. 30 and the tissue cutting device of FIG. 1;
FIG. 31B is a detail view of the distal tip of the trocar of FIG. 31A;
FIG. 32 is a side elevational view of an embodiment of a tissue imaging and cutting device;
FIG. 33A is a depiction of a surgeon performing an open craniotomy using a microscope and the tissue cutting device of FIG. 1;
FIG. 33B is a detail view of a portion of FIG. 31A;
FIG. 34A is a depiction of a surgeon performing an open craniotomy using an endoscope and the tissue cutting device of FIG. 1; and
FIG. 34B is a detail view of a portion of FIG. 32A.
FIG. 35 is a perspective view of a tissue cutting device with an angled handpiece in accordance with a second embodiment;
FIG. 36 is a cross-sectional view of the tissue cutting device of FIG. 35 depicting an inner cannula in a first relative position with respect to an outer cannula in which the inner cannula's distal end is located proximally of the outer cannula's distal end;
FIG. 37 is a cross-sectional view of the tissue cutting device of FIG. 35 depicting the inner cannula in a second relative position with respect to the outer cannula in which the inner cannula's distal end is located at the distal end of the outer cannula;
FIG. 38 is an exploded assembly view of the tissue cutting device of FIG. 35;
FIG. 39a is a side elevation view of a cam of the tissue cutting device of FIG. 35;
FIG. 39b is an end elevation view of the cam of FIG. 39a;
FIG. 40 is diagram of the tissue cutting device of FIG. 35 and the motor control unit of FIG. 22;
FIG. 41 is a partial cross-sectional view of the tissue cutting device of FIG. 35 depicting motor shaft position sensors for controlling a stop position of an inner cannula;
FIG. 42A depicts an exemplary method of using the tissue cutting device of FIG. 35 to perform a microscope assisted neurosurgical procedure;
FIG. 42B is a detail view of a portion of FIG. 42A;
FIG. 43 depicts a first exemplary method of gripping the tissue cutting device of FIG. 35 in a single hand;
FIG. 44 depicts a second exempalry method of gripping the tissue cutting device of FIG. 35 in a single hand;
FIG. 45 depicts a third exemplary method of gripping the tissue cutting device of FIG. 35 in a single hand;
FIG. 46 is a perspective view of a foot actuator assembly for use with a tissue cutting system;
FIG. 47 is a perspective view of the foot actuator assembly of FIG. 46 with a portion of a foot pedal cut away;
FIG. 48 is a perspective view of an operator's console for use with a tissue cutting system; and
FIG. 49 is a wiring diagram of a tissue cutting system including the foot actuator assembly of FIG. 46 and the console of FIG. 48.

### DETAILED DESCRIPTION

Referring now to the discussion that follows and also to the drawings, illustrative approaches to the disclosed systems and exemplary methods are shown in detail. Although the drawings represent some possible approaches, the drawings are not necessarily to scale and certain features may be exaggerated, removed, or partially sectioned to better illustrate and explain the present disclosure. Further, the descriptions set forth herein are not intended to be exhaustive or otherwise limit or restrict the claims to the precise forms and configurations shown in the drawings and disclosed in the following detailed description.

Described herein are tissue cutting devices that are suited for neurosurgical applications such as the removal of spine and brain tissue. The devices are configured with a "gun sight" design in which the axis of tissue cutting is spaced apart from a motor drive and drive assembly housing. The gun sight design provides a shorter device that is particularly well suited for many space-limited neurosurgical procedures.

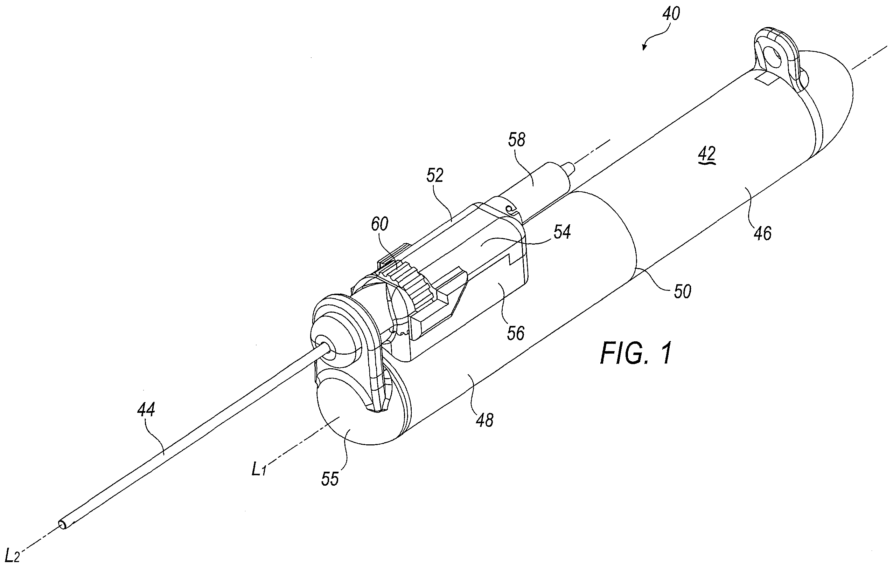

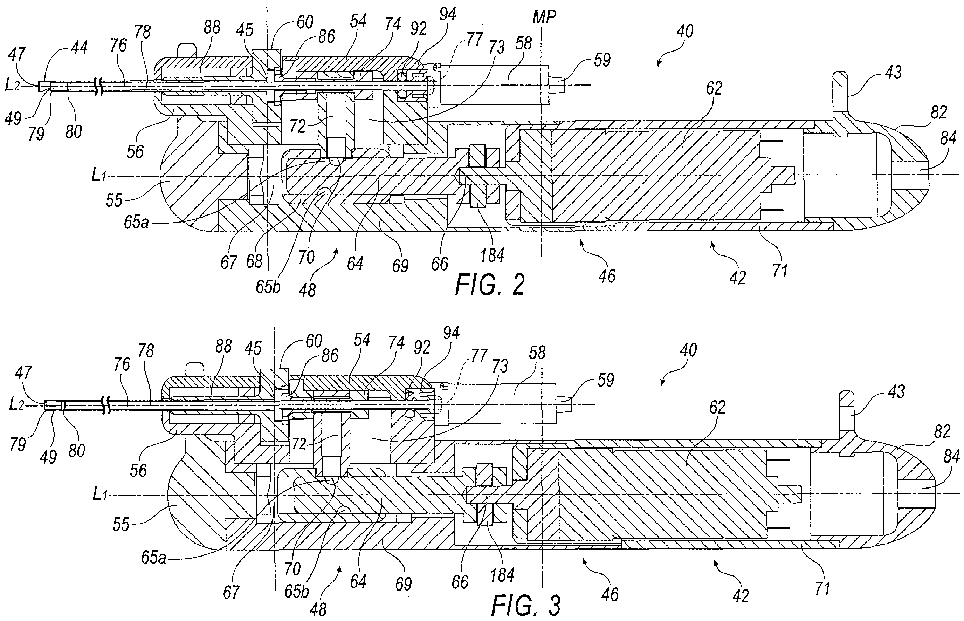

Referring to FIG. 1, a tissue cutting device 40 includes a handpiece 42 and an outer cannula 44. In one exemplary configuration, handpiece 42 is generally cylindrical in shape and is preferably sized and shaped to be grasped with a single hand. Handpiece 42 includes a lower housing 50 which comprises a proximal section 46 and distal section 48. Lower housing 50 has a longitudinal axis L₁ extending along its lengthwise direction. In addition, lower housing 50 has a middle point MP that is mid-way (FIGS. 2 and 3) between its proximal and distal ends.

Lower housing 50 comprises a proximal-most housing portion 82 (FIGS. 2 and 3) that is connected to a motor housing 71, and a cam housing 69 that is connected to motor housing 71. A front housing section 55 is connected to cam housing 69. Upper housing 52 is also provided. Upper housing 52 is connected to the lower housing 50 and is spaced apart from lower housing longitudinal axis L₁ in a direction that is perpendicular to L₁. In the example of FIGS. 2 and 3, upper housing 52 is located distally of the lower housing midpoint MP in the direction along the longitudinal axis L₁. A tissue collector 58 may be operatively connected to upper housing 52 (as will be explained in further detail below). A rotation dial 60 for rotating the outer cannula 44 with respect to handpiece 42 is also mounted to upper housing 52. Upper housing 52 is a generally elongated structure extending along a portion of the length of lower housing 50 and has a longitudinal axis L₂ which is spaced apart from and substantially parallel to lower housing axis L₁.

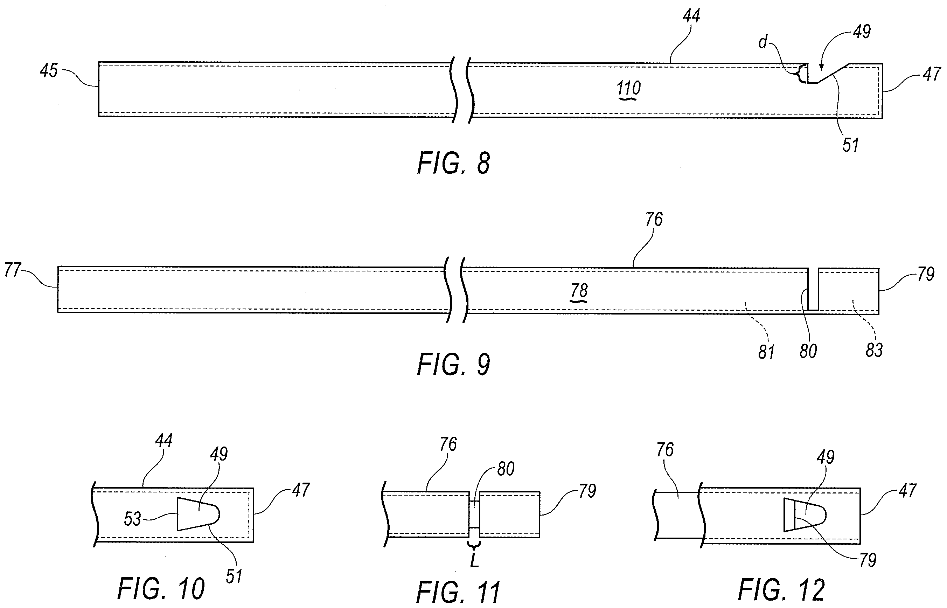

As best seen in FIGS. 2, 3, and 20, outer cannula 44 includes an open proximal end 45, a closed distal end 47, and a distal opening 49 proximate distal end 47. Outer cannula 44 is partially disposed in upper housing 52 and projects distally away from upper housing 52 in the direction of upper housing longitudinal axis L₂. Tissue cutting device 40 further comprises an inner cannula 76 which is partially disposed in an outer cannula lumen 110. Inner cannula 76 is configured to reciprocate within outer cannula lumen 110 (FIG. 8) and to cut tissue samples entering outer cannula 44 via outer cannula distal opening 49, as will be described in greater detail below. Inner cannula 76 extends along the longitudinal axis L₂ of the upper housing. In the example of FIGS. 2 and 3, inner cannula 76 is coaxial with upper housing longitudinal axis L₂. Inner cannula 76 reciprocates between a proximal position, which is depicted in FIG. 2, and a distal position which is depicted in FIG. 3. Inner cannula 76 includes an open proximal end 77 and an open distal end 79. Distal end 79 is preferably configured to cut tissue, and in preferred embodiments is capable of cutting neurological system tissues such as those from the brain or spine. In one exemplary embodiment, inner cannula distal end 79 is beveled in a radially inward direction to create a sharp circular tip and facilitate tissue cutting.

Outer cannula 44 is not translatable, and its position with respect to handpiece 42 along the direction of the longitudinal axes L₁ and L₂ remains fixed. Motor 62 is disposed in proximal lower housing section 46 of handpiece 42 and is operably connected to inner cannula 76 to drive the reciprocation of inner cannula 76 within outer cannula lumen 110 (FIG. 8). Motor 62 may be a reciprocating or rotary motor. In addition, it may be electric or hydraulic. However, in the embodiment of FIGS. 2 and 3, motor 62 is a rotary motor, the rotation of which causes inner cannula 76 to reciprocate within outer cannula lumen 110.

Motor 62 is housed in motor housing 71, which defines a portion of lower housing proximal section 46. Motor 62 is connected to an inner cannula drive assembly 63 (not separately shown in the figures) which is used to convert the rotational motion of motor 62 into the translational motion of inner cannula 76. At its proximal end, motor housing 71 is connected to proximal-most housing portion 82, which includes a power cable port 84 and a hose connector 43, which in the exemplary embodiment of FIG. 3 is an eyelet. Hose connector 43 provides a means of securely retaining a vacuum system hose to handpiece 42, thereby allowing vacuum to be supplied to tissue collector 58.

Inner cannula drive assembly 63 comprises a cam 64, a cam follower 68, a cam transfer 72, and a cannula transfer 74. As best seen in FIGS. 2 and 3, drive assembly 63 is spaced apart from upper housing 52, inner cannula 76, and outer cannula 44 in a direction that is substantially perpendicular to upper housing longitudinal axis L₂. By configuring device 40 with the cannulae 44 and 75 offset from lower housing 50 in this manner, the overall length of device 40 is reduced relative to the length of a fully "in-line" device in which the cannulae 44 and 76 are co-axially aligned with the drive assembly 63. The reduction in length provides a device 40 that is particularly well-suited for use in space-limited surgical procedures, such as many neurosurgical procedures.

In one illustrative example, device 40 has a weight distribution along the longitudinal axis L₁ such that the amount of weight between motor 62 and the proximal end 77 of inner cannula is greater than the amount of weight in motor housing 71 or distally of inner cannula proximal end 77 along the direction of axis L₁. In another example, device 40 has a center of gravity that is located distally of lower housing mid-point MP, and which his more preferably also proximal of inner cannula proximal end 77. The concentration of the device weight in the middle region of the device (i.e., between the proximal and distal ends) provides for a more ergonomic balance of the instrument which reduces user fatigue, allows for more accurate placement of the device during a procedure as well as more control over the device when it is required for more delicate and precise aspects of the procedure, such as when shaving tissue next to delicate or critical structures. The capability to have a device that is easier to handle and provides for a more balanced instrument reduces the amount concentration/focus and energy required from the surgeon and enables the surgeon to remove more tissue safely than prior art instrumentation., particularly when the device is used in a single-hand grip procedure.

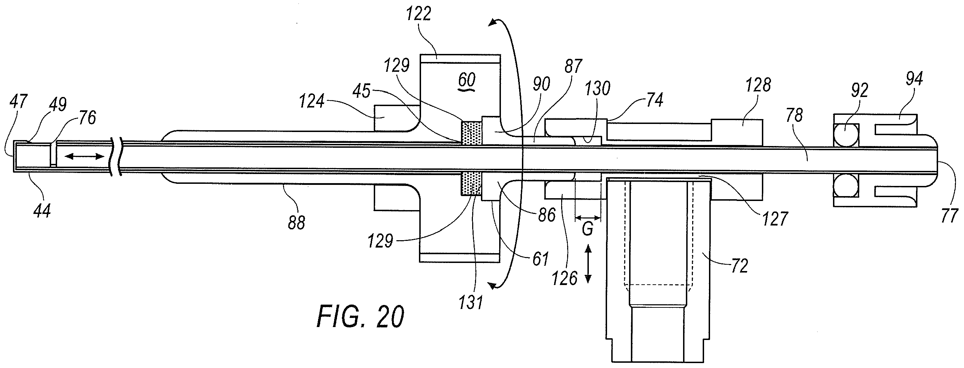

In addition, in one exemplary configuration, drive assembly 63 is located distally of lower housing midpoint MP such that it is disposed between the midpoint MP and the distal end of the lower housing 50. Drive assembly 63 is also positioned between the lower housing midpoing MP and the portion of outer cannula 44 that projects distally away from upper housing 52 with respect to the direction along the longitudinal axis L₁. In the example of FIGS. 2 and 3, drive assembly 63 is also located proximally of the proximal end 45 (best seen in FIG. 20) of outer cannula 44 such that drive assembly 63 is positioned between proximal outer cannula end 45 and lower housing midpoint MP along the direction of longitudinal axis L₁.

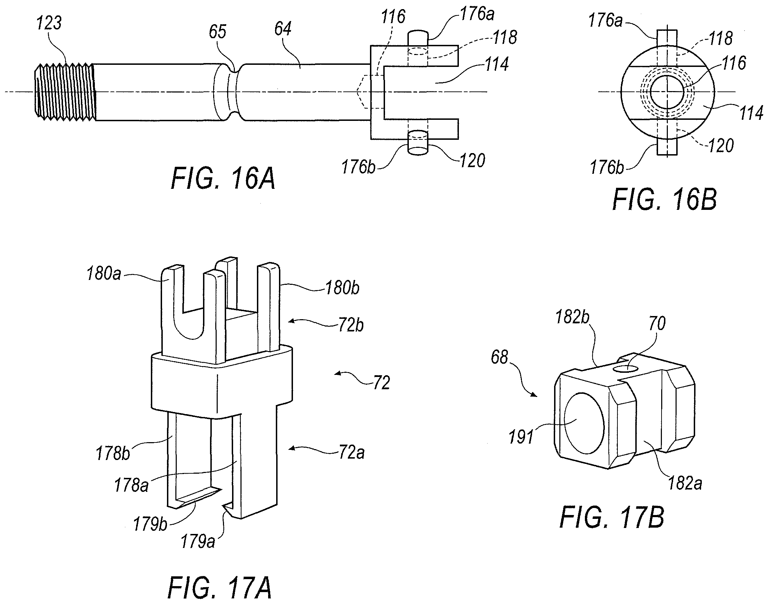

Cam 64 is a generally cylindrical structure and is shown in detail in FIGS. 16A and 16B. A groove or channel 65 is defined in the surface of cam 64. In one exemplary embodiment, groove 65 is continuous and circumscribes the perimeter of cam 64 but is not oriented perpendicularly to the longitudinal axis of cam 64, i.e., groove 65 is angled with respect to the cam axis. Opposing points on groove 65 such as points 65a and 65b (FIGS. 2 and 3) define pairs of "apexes" that are spaced apart along the longitudinal axis of the cam, i.e., the groove extends along a portion of the length of the cam. Cam 64 also includes a proximal opening 114 (FIG. 16a) for receiving a motor shaft and a proximal recess 116 into which a shaft may be snugly received. Holes 118 and 120 (FIGS. 16a and 16b) are provided for mounting cam rotational position indicators 176a and 176b that cooperate with a position sensor to determine the angular position of cam 64, and correspondingly, the linear position of inner cannula 76 within the outer cannula lumen 110, as discussed below.

Cam follower 68 is depicted in detail in FIG. 17B. Cam follower 68 is a generally rectangular block shaped structure with a hollow interior in which cam 64 is partially disposed. Cam follower 68 also includes a hole 70 in its upper face in which a ball bearing (not shown) is seated. The ball bearing rides in cam groove 65 and engages cam transfer 72. As a result, when cam 64 rotates about lower housing longitudinal axis L₁, cam follower 68 translates along the length of handpiece 42 in the direction of L₁. Cam follower 68 also includes lateral slots 182a and 182b that cooperatively engage corresponding members 178a, 178b from cam transfer 72.

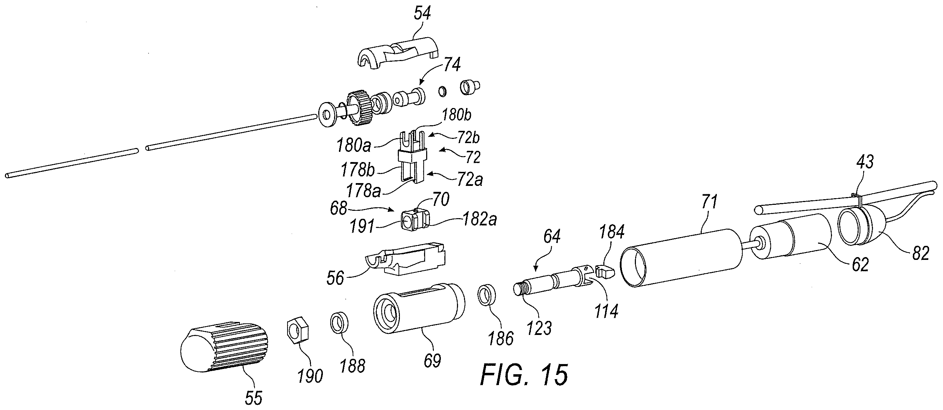

Cam follower 68 is disposed within a cam chamber 67 formed in cam housing 69 and is located distally of lower housing midpoint MP. Cam 64 is partially disposed in cam chamber 67 (FIGS. 2 and 3) and extends proximally therefrom to engage motor 62. Cam housing 69 comprises part of distal housing section 48 of handpiece 42. Cam 64 does not reciprocate within cam chamber 67 and instead merely rotates about its own longitudinal axis L₁. However, cam follower 68 reciprocates within cam chamber 67 along the direction of the longitudinal axis L₁. Cam follower 68 is open at its proximal end to receive cam 64. As shown in FIGS. 15 and 16A, cam 64 may optionally include a threaded distal end 123 that projects through a distal opening 191 (FIG. 17b) in cam follower 68 and which engages a nut 190 (FIG. 15) to prevent reciprocation of cam 64 relative to cam housing 69. Proximal cam bearing 186 and distal cam bearing 188 (FIG. 15) may also be provided to support cam 64 as it rotates within cam housing 69.

Cam transfer 72 extends from cam chamber 67 into a cam transfer chamber 73 formed in upper housing 52. As best seen in FIG. 17a, cam transfer 72 comprises a proximal end 72a that is attachable to cam follower 68 and a distal end 72b that is attachable to inner cannula 76 via cannula transfer 74. Proximal end 72a comprises a pair of spaced apart, downwardly extending members 178a and 178b, and distal end 72b comprises a pair of spaced apart upwardly extending members 180a and 180b. Downwardly extending members 178a and 178b are spaced apart in a direction that is perpendicular to lower housing longitudinal axis L₁, the length of cam 64, and handpiece 42, while upwardly extending members 180a and 180b are spaced apart in a direction that is parallel to the length of cam 64 and handpiece 42. Cam follower slots 182a and 182b engage downwardly extending members 178a and 178b of cam transfer 72. Downwardly extending members 178a and 178b of cam transfer 72 may be resilient and may have engagement portions 179a and 179b on their free ends (e.g., hooks or clips) for securely engaging the bottom and side surfaces of cam follower 68.

As best seen in FIG. 20, cannula transfer 74 comprises a sleeve disposed about inner cannula 76. Cannula transfer 74 comprises a proximal end 128, middle section 127, and distal end 126. Upwardly extending members 180a and 180b of cam transfer 72 (FIG. 17A) define fork-shaped structures that receive and cradle middle section 127 of cannula transfer 74. Distal end 126 and proximal end 128 of cannula transfer 74 are disposed outwardly of upwardly extending members 180a and 180b and are shaped to prevent relative translation between cam transfer 72 and cannula transfer 74. In the depicted embodiments, distal end 126 and proximal end 128 of cannula transfer 74 are enlarged relative to middle section 127 to abut the upwardly extending, fork-shaped members 182a and 182b, thereby preventing relative translation between cam transfer 72 and cannula transfer 74. As a result, when cam transfer 72 reciprocates along the direction of lower housing longitudinal axis L₁, cannula transfer 74 reciprocates as well. Because it is affixed to inner cannula 76, when cannula transfer 74 reciprocates, it causes inner cannula 76 to reciprocate within outer cannula 44 along the direction of upper housing longitudinal axis L₂.

In one exemplary arrangement, motor 62 is a brushed DC motor and may be operably connected to cam 64 in a number of ways. In the embodiment of FIGS. 2 and 3, motor 62 includes a distally extending shaft 66 that extends into a proximal opening 114 and engages recess 116 defined in cam 64 (FIG. 16A). Shaft 66 may be connected to cam 64 via a threaded connection, adhesive, or other known connection means. In an alternate implementation, depicted in FIG. 15, a separate cam coupler 184 is provided. Cam coupler 184 is seated in proximal opening 114 and has a width greater than the diameter of opening 114. Cam coupler 184 is also connected to motor shaft 66 such that rotation of shaft 66 causes cam coupler 184 to rotate, which in turn causes cam 64 to rotate therewith. One revolution of motor shaft 66 causes cam 64 to rotate by one revolution, which in turn causes inner cannula 76 to reciprocate by one complete stroke, i.e., from the position of FIG. 2 to the position of FIG. 3 and back to the position of FIG. 2.

Cam transfer 72 may be connected to cam follower 68 by mechanical means, adhesive means or other known connection means. In one exemplary embodiment, downwardly extending members 178a and 178b mechanically clip onto and removably engage cam follower 68. In another embodiment, cam transfer 72 is adhesively affixed to cam follower 68. In yet another embodiment, both mechanical and adhesive connections are used. The ball bearing (not shown) disposed in cam follower hole 70 traverses cam groove 65 as cam 64 rotates, causing cam follower 68 to reciprocate from the proximal position of FIG. 2 to the distal position of FIG. 3. As a result, cam transfer 72, cannula transfer 74 and inner cannula 76 translate between their respective proximal positions of FIG. 2 and their respective distal positions of FIG. 3 when motor 62 and cam 64 rotate. In certain examples (not separately shown), motor 62 may be connected to a cam follower, and the cam follower may be connected to a cam which is in turn operatively connected to the inner cannula. In accordance with these examples, when the motor rotates, the cam follower rotates and causes the cam to reciprocate, thereby causing the inner cannula to reciprocate.

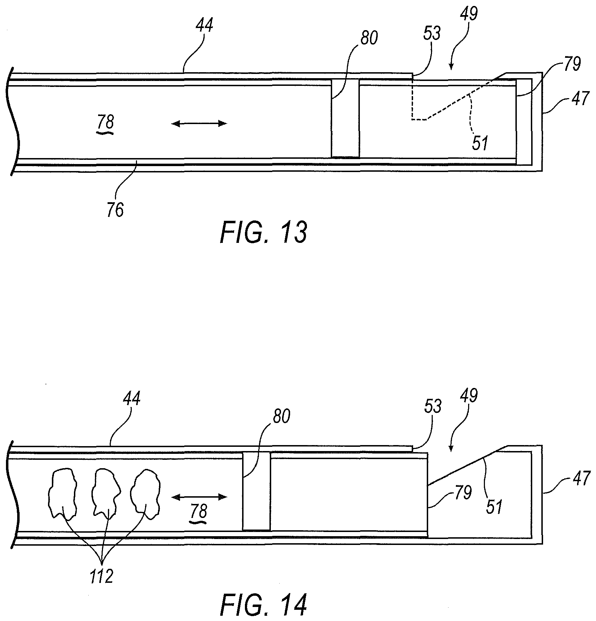

Motor 62 is preferably selected to have a rotational speed that allows inner cannula 76 to reciprocate from the position of FIG. 2 to the position of FIG. 3 and back to the position of FIG. 2 at a rate of at least about 1,000 reciprocations/minute. Reciprocation rates of at least about 1,200 reciprocations/minute are more preferred, and reciprocation rates of at least about 1,500 reciprocations/minute are even more preferred. Reciprocation rates of less than about 2,500 reciprocations/minute are preferred. Reciprocation rates of less than about 2,000 are more preferred, and reciprocation rates of less than about 1,800 reciprocations/minute are even more preferred. As best seen in FIG. 14, the rates of reciprocation of device 40 allow tissue to be severed into "snippets" 112 which are relatively smaller than "slug" tissue samples obtained by many prior devices. As the reciprocation continues, a continuum of severed tissue snippets 112 is obtained.

As mentioned previously, outer cannula 44 includes an opening 49 for receiving tissue into outer cannula lumen 110. As best seen in FIGS. 8-12, opening 49 is preferably defined by a cutting edge 51 that is configured to sever tissue and a non-cutting edge 53 that is not configured to sever tissue. In certain exemplary implementations, non-cutting edge 53 has a radial depth "d" that is no greater than about 50% of the outer diameter of outer cannula 44. In one exemplary implementation, cutting edge 51 is beveled in a radially inward direction, non-cutting edge 53 is not beveled, and cutting edge 51 is located immediately distally of non-cutting edge 53. Inner cannula distal end 79 is preferably configured to cut tissue. In one exemplary embodiment, distal end 79 is beveled in a radially inward direction around the circumference of inner cannula 76 to provide a sharp edge. As tissue is received in outer cannula opening 49, it is compressed between inner cannula distal end 79 and outer cannula cutting edge 51, causing the received tissue to be severed from the surrounding tissue.

Tissue cutting device 40 is particularly well suited for use in cutting tough tissues such as spinal and brain tissues. Outer cannula 44 and inner cannula 76 comprise materials that are generally rigid, such as rigid plastics or metal. In one preferred implementation, both cannulae comprise stainless steel, and more preferably, 304SS typically used in medical grade instruments.

As best seen in FIGS. 9-13, to facilitate the cutting of tough tissues, inner cannula 76 includes a hinge 80. Hinge 80 is located between inner cannula body section 81 which is located on the proximal side of hinge 80 and inner cannula cutting section 83 which is located on the distal side of hinge 80. In one exemplary arrangement, hinge 80 is a living hinge. As used herein, the term "living hinge" refers to a thin, flexible hinge that joins two relatively more rigid parts together. In one example, hinge 80 is a living hinge that is integrally formed with inner cannula body section 81 and inner cannula cutting section 83 by removing a portion of the circumference of the inner cannula 76 along a length L (FIG. 11). Hinge 80 allows cutting section 83 to pivot about hinge 80 as inner cannula 76 reciprocates within outer cannula 44. As inner cannula 76 translates in the distal direction, it contacts tissue received in outer cannula opening 49 and encounters progressively increasing resistance from the tissue as the tissue is urged in the distal direction. As the resisting force of the tissue increases, cutting section 83 pivots progressively more until a zero annular clearance is obtained between inner cannula distal end 79 and outer cannula opening 49. The received tissue is severed and aspirated in the proximal direction along inner cannula lumen 110 and received in tissue collector 58. Thus, inner cannula lumen 110 provides an aspiration path from the inner cannula distal end 79 to the inner cannula proximal end 77. Hinge 80 allows a generally zero annular clearance to be obtained between inner cannula distal end 79 and outer cannula opening 49 at cutting section 83 while not affecting the annular clearance between inner cannula body section 81 and outer cannula 44. This configuration maximizes tissue cutting while minimizing frictional losses that would otherwise occur due to the frictional engagement of the outer surface of inner cannula body section 81 and the inner surface of outer cannula 44 if a very small annular clearance between the outer cannula 44 and inner cannula 76 were present.

Outer cannula opening 49 may have a number of shapes. In certain examples, when outer cannula opening 49 is viewed in plan, it has a shape that is generally square, rectangular, trapezoidal, ovular, or in the shape of the letter "D." In certain other exemplary implementations, outer cannula opening 49 is configured to direct tissue so that it may be compressed as inner cannula 76 translates in the distal direction. In one such implementation, depicted in FIGS. 10 and 12, outer cannula opening 49 has a generally triangular shape when outer cannula opening 49 is viewed in plan. As FIGS. 10 and 12 indicate, when viewed in plan, the width of opening 49 in a direction transverse to the outer cannula longitudinal axis L₂ varies longitudinally along the length of outer cannula 44, and preferably narrows from the proximal to distal portions of opening 49. When viewed in side elevation, cutting edge 51 slopes in a radially outward direction moving distally along edge 51 in the direction of longitudinal axis L₂. As a result, as a tissue sample is distally urged within outer cannula opening 49 by the action of inner cannula 76, the tissue is increasingly compressed in the direction of the circumference of inner cannula 76 (or in the direction of the "width" of opening 49 when viewed in plan). To ensure complete cutting, inner cannula distal end 79 preferably travels to a position that is distal of outer cannula opening 49 during a tissue cutting operation, i.e., there is an inner cannula overstroke.

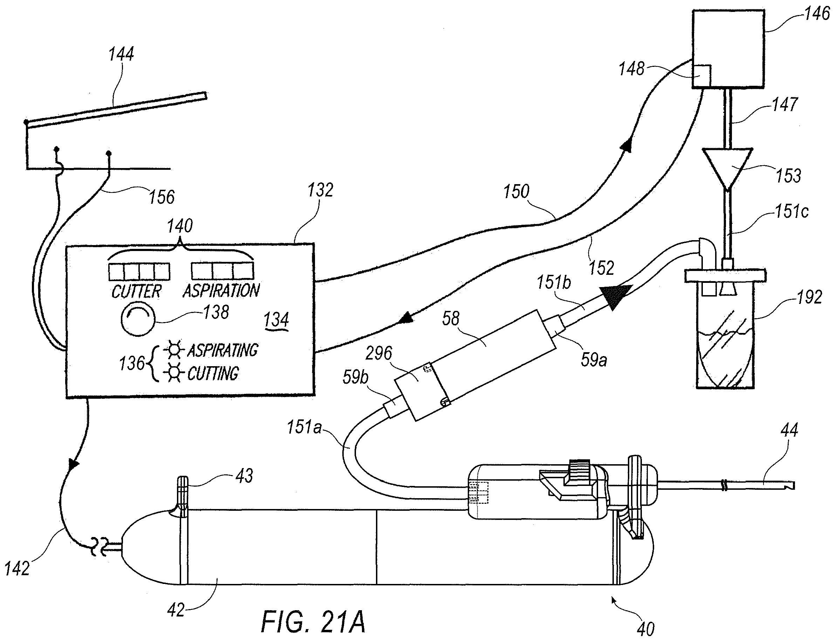

As mentioned above, tissue cutting device 40 aspirates tissue samples received in inner cannula lumen 78 to cause the tissue samples to move in the proximal direction along the length of the inner cannula 76. In certain exemplary methods of use, device 40 is used to resect tissue without collecting tissue samples for further analysis. In such embodiments, a tissue collector need not be provided. In other embodiments wherein tissue collection is desired, device 40 preferably includes a tissue collector 58 into which aspirated tissue samples are deposited during a tissue cutting procedure. Tissue collector 58 may be located remotely from handpiece 42 and outside the sterile field during a tissue cutting operation as shown in FIG. 21A. However, in an alternative embodiment, as best seen in the examples of FIGS. 1-7, tissue collector 58 is removably connected to handpiece 42. In either embodiment, a fluid collection canister 192 is preferably located between tissue collector 58 and a source of vacuum (such as vacuum generator 153 in FIG. 21A) to protect the vacuum generating apparatus from becoming contaminated or damaged by aspirated fluids. In those embodiments that lack a tissue collector, fluid collection canister 192 may be provided to collect both aspirated fluid and tissue.

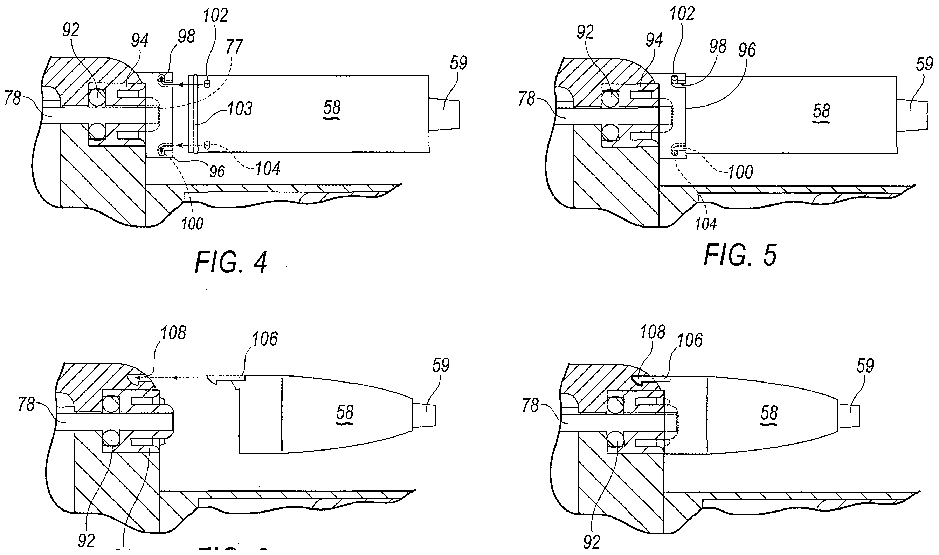

Referring to FIGS. 4-7, tissue collector 58 is connected to upper housing 52 proximally of the inner cannula 76 to receive the aspirated tissue samples. Tissue collector 58 is a generally cylindrical, hollow body with an interior volume that is in fluid communication with the inner cannula lumen 78 and a source of vacuum (not shown in FIGS. 4-7). Tissue collector 58 is removably secured to housing connector 96 to allow for the periodic removal of collected tissue samples. Tissue collector 58 is preferably secured to upper housing 52 in a manner that provides a substantially leak-proof vacuum seal to maintain consistent aspiration of severed tissue samples. A vacuum hose fitting 59 is formed on the proximal end of tissue collector 58 and is in fluid communication with the interior of tissue collector 58 and with a vacuum generator, as will be discussed below.

In the embodiment of FIGS. 4-5, housing connector 96 is a generally cylindrical, flange extending proximally from upper housing 52. Upper shell 54 and lower shell 56 of upper housing 52 cooperatively define a cavity into which a seal holder 94 is partially disposed. Seal holder 94 includes a distal annular recess in which a seal 92, such as an o-ring, is disposed. Seal holder 94 also includes a central lumen through which inner cannula 76 is slidably disposed. A proximally projecting portion 95 of seal holder 94 projects away from upper housing 52 in the proximal direction and is received within housing connector 96. As best seen in FIGS. 2 and 3, inner cannula proximal end 77 preferably remains within seal holder 94 as inner cannula 76 reciprocates during operation of tissue cutting device 40. However, proximal end 77 moves within seal holder 94 as inner cannula 76 reciprocates. Seal 92 preferably comprises a resilient material such as an elastomeric material. The sealing engagement of seal 92 and inner cannula 76 prevents air or fluids from leaking between inner cannula 76 and upper housing 52 and aids in maintaining consistent aspiration of samples through the inner cannula lumen 78.

Housing connector 96 includes connecting features 98 and 100 which are configured to engage with corresponding connecting features 102 and 104 on tissue collector 58. In the embodiment of FIGS. 4 and 5, connecting features 98 and 100 are "J" shaped slots formed in housing connector 96, and connecting features 102 and 104 are complementary protrusions formed on tissue collector 58 which engage connecting features 98 and 100, respectively. To connect tissue collector 58 to housing connector 96, protrusions 102 and 104 are aligned with slots 98 and 100, and tissue collector 58 is then inserted into housing connector 96 in the distal direction. Tissue collector 58 is then rotated to fully engage protrusions 102 and 104 with slots 98 and 100. A seal 103 is provided around the circumference of tissue collector 58 to sealingly engage the inner surface of housing connector 96.

An alternate embodiment of tissue collector 58 is depicted in FIGS. 6 and 7. In the embodiment of FIGS. 6 and 7, tissue collector 58 is semi-elliptical in cross-section and includes a hollow interior for receiving samples, as in the embodiment of FIGS. 4 and 5. In the embodiment of FIGS. 6 and 7, a cylindrical flange housing connector 96 is not provided. Instead, upper housing 52 is formed with an engagement recess 108 that engages a complementary clip 106 formed on tissue collector 58. In each of the foregoing embodiments, tissue collector 58 may be provided with a filter (not shown) in its interior for collecting solid tissue samples while allowing liquids and gases (e.g., air) to pass through. Exemplary filters include medical grade mesh filters with a mesh size smaller than that of tissue snippets 112.

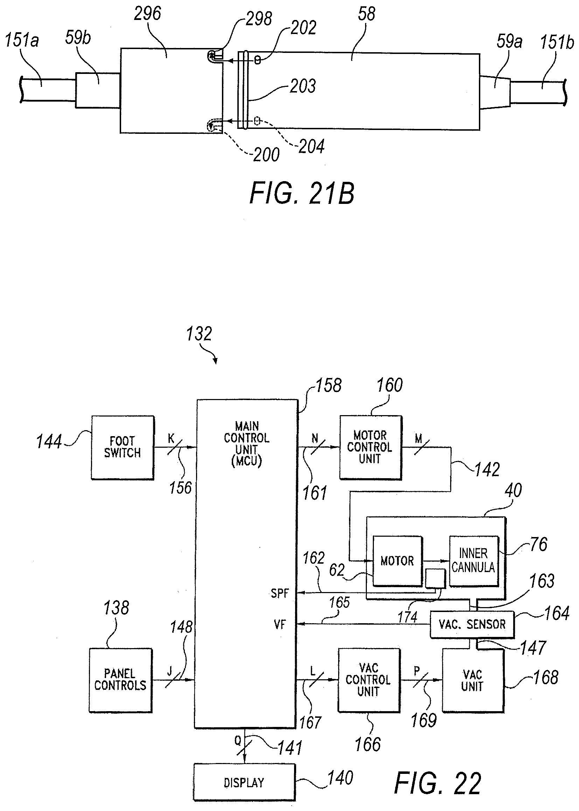

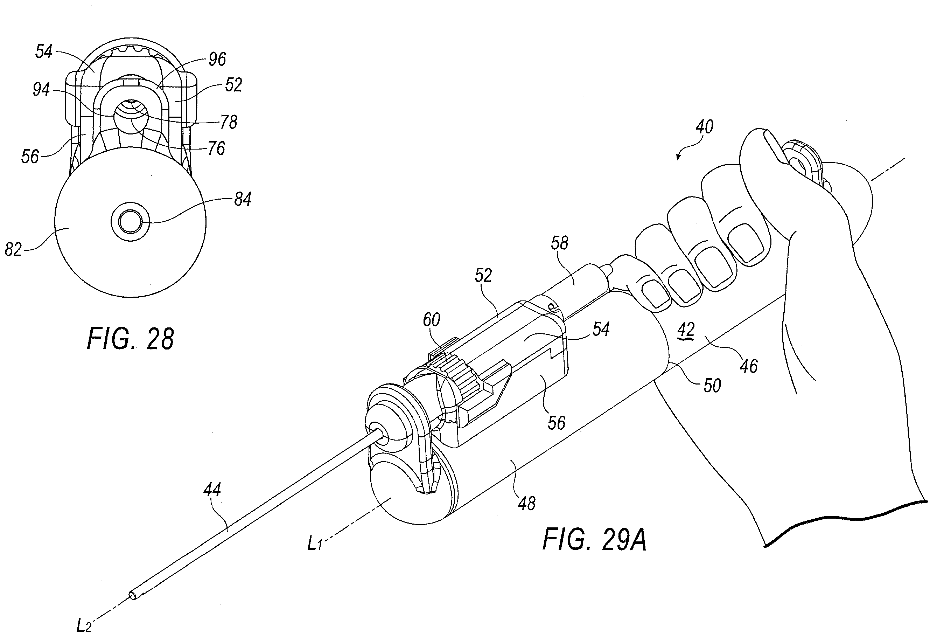

In the embodiments of FIGS. 4-7, tissue collector 58 preferably has a longitudinal axis that is not collinear with the longitudinal axis L₁ of handpiece lower housing 50, motor 62, and cam 64. The longitudinal axis of tissue collector 58 is preferably substantially collinear with the longitudinal axis L₂ of inner cannula 76 to yield an "in-line" filter configuration. Tissue collector 58 and inner cannula 76 are both spaced apart from and substantially parallel to the longitudinal axis L₁ of handpiece lower housing 50, motor 62, and cam 64. Thus, the cutting axis (i.e., the outer cannula longitudinal axis, which is collinear with upper housing longitudinal axis L₂) and sample aspiration path axis through inner cannula lumen 78 are not coaxial with the longitudinal axis L₁ of the handpiece lower housing 50. As a result, when device 40 is used to cut tissue, the surgeon's view of the cutting axis is not obstructed by his or her hand. As best seen in FIG. 28, the surgeon can treat the proximal end of the upper housing 52 and/or filter 58 as a "gun sight" and align it with a tissue sample to be cut to thereby align the outer cannula 44 with the tissue sample, providing enhanced ergonomic benefits over previous devices, in particular, previous neurosurgical devices. In the case of a device with a remote tissue collector 58 such as the one depicted in FIGS. 21A and 21B, the user can treat the proximal end of upper housing 52 as a gun sight and align it with a target tissue.

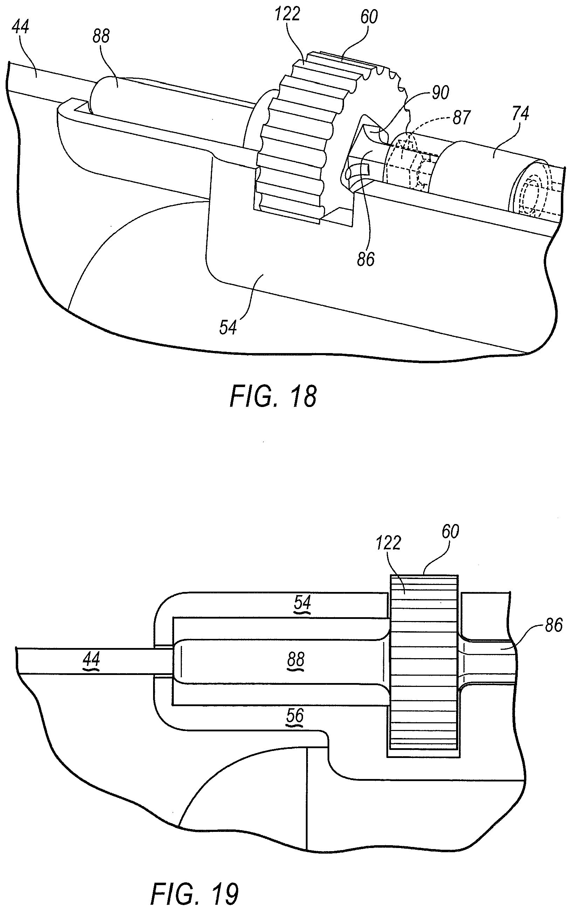

When device 40 is used to cut tissue, outer cannula opening 49 must be aligned with the target tissue of interest to receive it for cutting. The entire device 40 can be rotated about the longitudinal axis of handpiece 42 to place outer cannula opening 49 at the desired location. However, this technique can be awkward and may reduce the surgeon's dexterity. Thus, in an exemplary embodiment, device 40 includes a selectively rotatable outer cannula 44. As best seen in FIGS. 18-20, a rotation dial 60 is provided and is rotatably seated in a cavity defined by upper shell 54 and lower shell 56 of upper housing 52. Rotation dial 60 is configured such that when it is rotated, it causes outer cannula 44 to rotate about its longitudinal axis L₂. Rotation dial 60 is preferably connected to an outer cannula connector portion 88. In the embodiment of FIGS. 18-20, outer cannula connector portion 88 is a sleeve that is integrally formed with rotation dial 60 and which is fixedly secured to outer cannula 44 such as by an adhesive or other known connection means. In the exemplary embodiment of FIG. 20 rotation dial 60 has an outer diameter that is greater than that of outer cannula connector portion 88.

As mentioned previously, inner cannula 76 includes a hinge 80 to allow inner cannula cutting section 83 to pivot toward outer cannula opening 49 when device 40 is in operation. In order to ensure the correct operation of hinge 80, the circumferential alignment of hinge 80 and outer cannula opening 49 should be maintained. Thus, rotation dial 60 is preferably connected to inner cannula 76 such that when rotation dial 60 is rotated, both outer cannula 47 and inner cannula 76 rotate in a fixed angular orientation with respect to one another by an amount that directly corresponds to the amount by which rotation dial 60 is rotated. Rotation dial 60 may be directly connected to inner cannula 76 or may use an intervening connecting device. However, rotation dial 60 should be configured to allow inner cannula 76 to reciprocate with respect to rotation dial 60. As best seen in FIG. 20, rotation dial inner cannula connector 86 may be provided to connect rotation dial 60 to inner cannula 76. Rotation dial inner cannula connector 86 comprises a proximal sleeve 87 disposed about inner cannula 76 and a distal, radially extending annular flange 90 with an outer diameter greater than that of the sleeve 87. Sleeve 87 and flange 90 may be in the shape of circular cylinders. Alternatively, and as shown in FIGS. 18-19, sleeve 87 and flange 90 may be in the shape of a polygonal cylinder. Sleeve 87 is slidably received within the annular cavity 130 at the distal end 126 of the cannula transfer 74 and keyed to the inner surface of cannula transfer 74 at annular cavity 130 such that sleeve 87 can reciprocate with respect to cannula transfer 74 while causing cannula transfer 74 to rotate with sleeve 87 when rotation dial 60 is rotated. When inner cannula 76 is reciprocated, cannula transfer distal end 126 reciprocates with respect to sleeve 87, thereby variably adjusting gap "G" defined within annular cavity 130 (FIG. 20). Alternatively, cannula transfer distal end 126 may be slidably received in an annular cavity formed in sleeve 87 and may be keyed to the inner surface of the annular cavity so that cannula transfer may reciprocate with respect to sleeve 87 while still rotating with sleeve 87 when dial 60 is rotates.

As best seen in FIG. 20, rotation dial 60 includes a first annular cavity 61 that is sized to receive and engage flange 90 in a close fitting relationship. Rotation dial 60 may be press fit to flange 90. In addition, adhesive connections or mechanical connections may be used. Because rotation dial 60 is directly or indirectly connected to both outer cannula 44 and inner cannula 76, both cannulae rotate in direct correspondence to the rotation of rotation dial 60, thereby allowing the user to adjust the orientation of outer cannula opening 49 and inner cannula hinge 80 in a circumferential direction with respect to handpiece 42. As a result, surgeons need not rotate the entire tissue cutting device 40 to obtain the desired angular orientation.

Rotation dial 60, outer cannula 44, and inner cannula 76 are preferably configured for 360° rotation. In addition, tactile indicators are preferably provided on rotation dial 60 to allow a user to reliably determine the extent to which dial 60 has been rotated from a given starting point. The tactile indication may comprise surface features defined on or in the exterior surface of rotation dial 60. In one exemplary embodiment, depicted in FIGS. 18-20, a plurality of ridges 122 is provided around the circumference of rotation dial 60 to provide tactile indication. The ridges also act as grips and facilitate the surgeon's ability to rotate the dial 60 without transferring unwanted motion to the surgical site.

As mentioned previously, vacuum (sub-atmospheric pressure) is applied to tissue collector 58 to aspirate severed tissue samples through inner cannula 76 in the proximal direction. The application of vacuum to inner cannula 76 via tissue collector vacuum hose fitting 59 will have a propensity to produce a vacuum at proximal end 45 of outer cannula 44 if leakage occurs between inner cannula 76 and the components of upper housing 52. The generation of a vacuum at outer cannula proximal end 45 will in turn cause fluids and/or tissue samples at the distal end of outer cannula 44 to flow into the annular clearance between inner cannula 76 and outer cannula 44 that extends from its proximal end at outer cannula proximal end 45 to its distal end at inner cannula distal end 79. This fluid and/or tissue can result in blockage of the annular clearance and increased friction between the inner cannula 76 and outer cannula 44, resulting in degraded performance. Accordingly, a seal 129 is preferably provided to prevent air artifacts, fluid (water, saline, blood, etc.) flow, and tissue sample flow in the annular clearance between inner cannula 76 and outer cannula 44. The seal 129 is preferably disposed adjacent the proximal end of the annular clearance between inner cannula 76 and outer cannula 44, i.e., proximally adjacent to outer cannula proximal end 45. As shown in FIG. 20, seal 129 is preferably annular and circumscribes inner cannula 76, extending from the outer surface of inner cannula 76 in a radially outward direction as well as longitudinally along a portion of the length of inner cannula 76.

In the embodiment of FIG. 20, rotation dial 60 and sleeve 87 act as a seal housing and include a seal cavity 131 which is an annular cavity disposed immediately adjacent to and distal of first annular cavity 61. Seal cavity 131 is sized to accept seal 129 therein. The seal 129 may be a conventional seal such as a solid, flexible and/or elastomeric o-ring. However, seal 129 is preferably amorphous and comprises a thixotropic material that is a semi-solid. It is further preferred that seal 129 fill the entirety of seal cavity 131 to ensure that cavity 131 is substantially leak free. In the exemplary embodiment of FIG. 20, seal cavity 131 has an outer diameter that is greater than the outer diameter of outer cannula 44. Moreover, the annular thickness of seal cavity 131 is preferably greater than the annular clearance between outer cannula 44 and inner cannula 76 to better ensure complete sealing of the annular clearance.

In one exemplary embodiment, seal 129 is a grease--such as the so-called "high vacuum greases"--that is formulated to withstand vacuum conditions. Suitable high vacuum greases include halogenated polymers. The halogenated polymers are preferably based on cyclic ethers or unsaturated hydrocarbon polymeric precursors. In one exemplary embodiment, a perfluroropolyether (PFPE) grease is used. Examples of such greases include the FOMBLIN^{®} family of greases supplied by Solvay Solexis, Inc. Other examples of such greases include polytetrafluroroethylene greases ("PTFE") such as TEFLON^{®} greases supplied by DuPont. One suitable high vacuum grease is FOMBLIN^{®} Y VAC3 grease, which is a PFPE grease with a PTFE thickener. The semi-solid seal 129 preferably has a kinematic viscosity at 20°C of at least about 500 cSt, more preferably at least about 800 cSt, and even more preferably at least about 1200 cSt. Semi-solid seal 129 preferably has a kinematic viscosity at 20°C of no greater than about 2500 cSt, more preferably no greater than about 2000 cSt, and even more preferably no greater than about 1700 cSt.

The use of a semi-solid seal 129 has several advantages. Because the seal is semi-solid, it will tend to absorb and dampen vibrations transmitted from the reciprocation of the inner cannula, thereby reducing overall vibration of device 40, and in particular, the vibration transmitted to outer cannula 44. The dampening of such vibrations is particularly beneficial because it prevents unwanted vibration of outer cannula 44 which can disturb delicate neurosurgical procedures. Moreover, because it is not a solid seal, seal 129 will experience less heating and wear as it is frictionally engaged by the reciprocating inner cannula 76. In certain embodiments, a portion of seal 129 will adhere to the outer surface of inner cannula 76 as it reciprocates producing a zero slip velocity condition at the inner cannula 76 outer surface which may further reduce frictional heating and degradation of seal 129. Because semi-solid seal 129 produces less frictional resistance to the reciprocation of inner cannula 76 as compared to conventional solid seals such as o-rings, it also decreases the required motor power consumption and can facilitate the use of lower torque and lower cost motors, which in turn facilitates making device 40 disposable.

In one configuration, device 40 is connected to a vacuum source and configured for variable aspiration, i.e., configured to supply variable levels of vacuum to inner cannula lumen 78. As depicted in FIG. 21A, in one exemplary implementation, a tissue cutting system is provided which comprises tissue cutting device 40, a tissue collector 58, a controller 132, a vacuum generator 153, a vacuum actuator 144, a controllable valve 146, a vacuum line 151, and a fluid collection canister 192. As mentioned previously, in FIG. 21A tissue collector 58 is located remotely from handpiece 42 and may be placed far enough from the handpiece 42 to remain outside of the sterile field during a tissue cutting operation. As best seen in FIG. 21B, tissue collector 58 is generally the same as the tissue collector 58 depicted in FIGS. 4-5. Vacuum line 151a connects the distal end of tissue collector 58 to proximally projecting portion 95 of seal holder 94 on the proximal end of tissue cutting device upper housing 52. In one arrangement, the proximal end of vacuum line 151a includes a hose fitting 59b that is integrally formed with a tissue collector coupler 296. Coupler 296 is similar in structure to tissue collector connector 96 (FIGS. 4-5) and is a cylindrical structure with a hollow interior for receiving a portion of tissue collector 58. As best seen in FIG. 21B, tissue collector 58 includes projections 202 and 204 which engage complementary slots 298 and 200 in coupler 296 in the same manner that projections 102 and 104 engage slots 98 and 100 in FIGS. 4-5. At the proximal end of tissue collector 58, hose fitting 59a engages vacuum line 151b which in turn is connected to fluid collection canister 192. Fluid collection canister 192 is connected to vacuum generator 153 via vacuum line 151c. Vacuum generator 153 is connected to controllable valve 146 by way of pressure line 147.

The outlet of tissue collection canister 192 is preferably substantially liquid free and is connected to vacuum generator 153 via vacuum line 151c. Thus, vacuum generator 153 is in fluid communication with tissue collector 58 and inner cannula lumen 78, thereby generating a vacuum at the proximal end 77 of inner cannula 76 to aspirate severed tissue samples from inner cannula distal end 79 to tissue collector 58. The level of vacuum generated by vacuum generator is preferably variable and selectively controllable by a user. Maximum vacuum levels of at least about 0 in Hg. are preferred, and maximum vacuum levels of at least about 1 in Hg. are more preferred. Maximum vacuum levels of at least about 5 in Hg. are even more preferred, and maximum vacuum levels of at least about 10 in Hg. are still more preferred. Maximum vacuum levels of at least about 20 in. Hg. are yet more preferred, and vacuum levels of at least about 29 in. Hg. are most preferred.

The controllable valve 146 and the vacuum generator 153 provide a means for continuously adjusting and controlling the level of vacuum applied to tissue collector 58 and the proximal end of inner cannula lumen 78. Controllable valve 146 is supplied with a pressurized gas, preferably air, or an inert gas such as nitrogen. In one exemplary embodiment, the pressure applied to controllable valve 146 is about 70 psi.

The system further includes an electrical controller 132 which receives and provides signals to the various components to control or monitor their operations. Controller 132 provides control signals to device 40 via motor drive control line signal path 142 to activate or deactivate motor 62. An aspiration valve control line 150 extends from the controller 132 to the controllable valve 146 which provides pressure to the vacuum generator 153. Signals to the controllable valve 146 through line 150 are used to control the amount of vacuum applied to tissue collector 58.

Controller 132 also receives electrical signals from the various components of the system. For instance, a pressure transducer 148 associated with the aspiration controllable valve 146, sends a signal along line 152 to the controller 132. The signal is representative of the pressure supplied through controllable valve 146 to vacuum generator 153. Thus, the transducer 148 provides immediate feedback to the controller which can in turn provide signals to aspiration controllable valve 146.

The user can adjust the system operating parameters by using panel controls such as a console knob 138 and/or one or more depressible controllers, such as a foot pedal 144. In one embodiment, foot pedal 144 can be used to activate the motor 62 in device 40, causing the inner cannula 76 to reciprocate within the outer cannula 44. In another embodiment, foot pedal 144 can be used to control the vacuum level supplied from vacuum generator 153 to tissue collector 58 and inner cannula lumen 78. In another embodiment, foot pedal 144 can be used both to activate motor 62 and to control the vacuum level supplied from vacuum generator 153 to tissue collector 58. In one arrangement foot pedal 144 is configured to variably increase the level of vacuum applied to tissue collector 58 from a minimum level to a maximum level as foot pedal 144 is depressed from a first position to a second position. In such an arrangement, the first position is one in which foot pedal 144 is not depressed at all or is only slightly depressed, and the second position is one in which foot pedal 144 is fully depressed. In another embodiment, knob 138 is used to set a preselected maximum vacuum level applied by vacuum generator 153. Thus, by depressing foot pedal 144 from a first fully open position to a second fully closed position, a plurality (preferably a continuum) of vacuum levels can be supplied to tissue collector 58 with the maximum vacuum level being user adjustable via knob 138.

In one exemplary embodiment, foot pedal 144 includes two switches (not shown) for providing variable vacuum and activating motor 62. In one exemplary embodiment, once foot pedal 144 is partially depressed from an open or undepressed position, motor 62 is activated. In accordance with the embodiment, continued depression of foot pedal 144 activates vacuum generator 153. Foot pedal 144 preferably provides continuous movement between a fully open and a fully depressed position which in turn corresponds to a plurality, and preferably a continuum, of vacuum levels that are supplied to inner cannula lumen 78. Once foot pedal 144 is fully depressed, the vacuum level supplied to inner cannula lumen 78 corresponds to a previously selected maximum vacuum level.

In certain illustrative examples, the user will adjust the level of vacuum to achieve a desired level of "traction" in the tissue surrounding the tissue to be severed. As used here in, the term "traction" refers to the exertion of a pulling force on tissue surrounding the target tissue to be severed. In some instances, traction may be visualizable by the surgeon with the use of a magnification instrument, such as a microscope or an endoscope. The level of vacuum will also determine the amount of unsevered tissue that is drawn into outer cannula opening 49, and therefore, the size of the severed tissue snippets 112 (FIG. 14). Therefore, when fine shaving operations are desired, the vacuum level will be a relatively lower level than if debulking (large scale tissue removal) is performed. Of course, the pre-selected maximum vacuum level will also affect the maximum size of tissue that is drawn into outer cannula opening 49, and therefore, will affect the maximum size of severed tissue samples during any one operation. Also, the vacuum level may be adjusted based on the elasticity, fibrotic content, and hardness/softness of the tissue.

Console 134 may also include indicator lights 136, one of which indicates the activation of cutting and one of which indicates the activation of aspiration. Console 134 may further include an analog display 140 with readouts for "aspiration" and "cutter." The "aspiration" read out indicates the vacuum level supplied to tissue collector 58 from vacuum generator 153. The "cutter" read out indicates the speed of reciprocation of inner cannula 76. In one embodiment, a speed sensor is mounted in device 40 to determine the speed of reciprocation of inner cannula 76 and the sensor is input to controller 132.

As mentioned previously, when device 40 is used to perform a cutting operation, inner cannula 76 reciprocates within outer cannula opening 49 to sever tissue received within outer cannula opening 49. When a cutting operation is complete, it may be preferred to have inner cannula 76 come to rest at a position that is proximal of the non-cutting edge 53 of outer cannula opening 49 to ensure that tissue is not trapped between inner cannula distal end 79 and outer cannula cutting edge 51. However, in certain exemplary methods of use, tissue cutting device 40 may be used as an aspiration wand without cutting any tissue. In these embodiments, the stop position of the inner cannula distal end 79 within outer cannula opening 49 determines the open area of the outer cannula 44, and therefore, the aspiration levels that can be applied immediately adjacent outer cannula opening 49. Thus, in some preferred embodiments, the inner cannula stop position is user adjustable. Tissue cutting device 40 may be used to aspirate a variety of fluids associated with a neurosurgical procedure, including without limitation blood, saline, cerebrospinal fluid, and lactate ringer's solution. In certain examples, the inner cannula stop position is adjusted to provide a desired degree of aspiration, outer cannula 44 is positioned proximate a target tissue, and vacuum is applied to manipulate the target tissue and draw it into outer cannula opening 49. Outer cannula 44 is then moved to a desired location or orientation, thereby moving the target tissue to the desired location or orientation. Once the target tissue has been satisfactorily manipulated, a cutting operation is initiated. By using device 40 in this manner, target tissues can be drawn away from areas where tissue cutting operations are undesirable, and the cutting can be performed remotely from those areas.

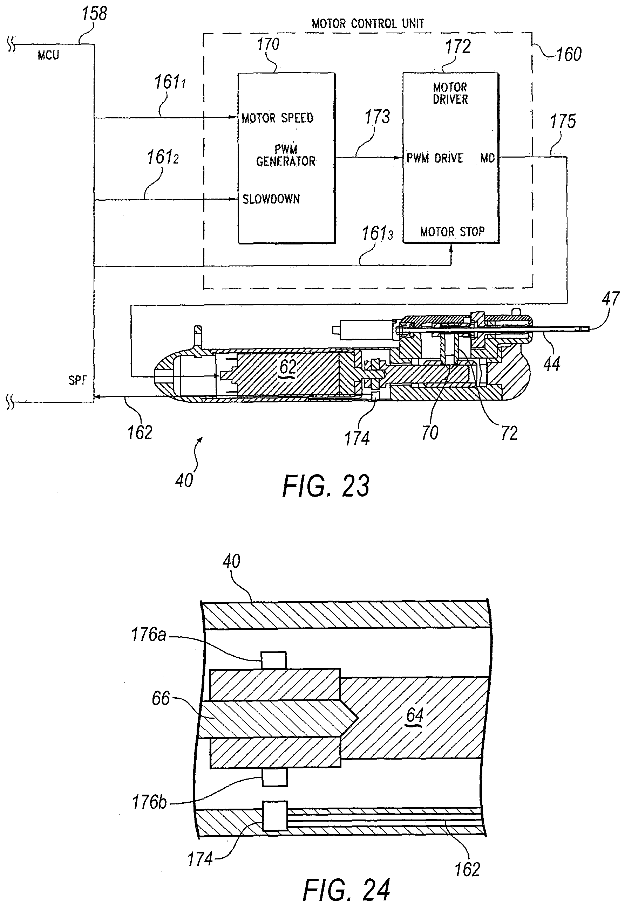

In one exemplary system, an inner cannula position control is provided which controls the rest position of inner cannula 76 when motor 62 is deactivated. Referring to FIG. 24, cam rotational position indicators 176a and 176b are mounted on the proximal end of cam 64. In an exemplary embodiment; cam rotational position indicators 176a and 176b are magnets having opposite poles. A position sensor 174 is mounted on the inner surface of cam housing 69 and generates a signal indicative of the rotational position of indicators 176a and 176b relative to position sensor 174. As mentioned previously, the rotation of cam 64 correlates directly to the position of inner cannula 76 within outer cannula 44. Thus, the rotation of cam 64 can be sensed to indirectly determine the position of inner cannula 76. Accordingly, indicators 176a/176b and sensor 174 can be used to determine the position of inner cannula 76 with respect to non-cutting edge 53 of outer cannula opening 49 (FIGS. 10-12).

Referring to FIG. 22, an embodiment of a system for controlling the operation of tissue cutting device 40 is provided. The system includes a main control unit 158 ("MCU"), which (in the embodiment shown) is configured as a microprocessor-based system. In one implementation, MCU 158 is incorporated in controller 132 and is operable to control the various operations of the tissue cutting device 40. Foot switch 144 is electrically connected to a number of inputs of MCU 158 via an equal number, K, of signal paths 156, wherein K may be any integer. Panel controls, such as knob 138, are electrically connected to a number of inputs of MCU 158 via an equal number, J, of signal paths 145, wherein J may be any integer.

Display unit 140 is electrically connected to a number of outputs of MCU 158 via an equal number, Q, of signal paths 141, wherein Q may be any integer. In one exemplary implementation, depicted in FIG. 21A, display unit 140 is provided on console 134.

As mentioned previously, tissue cutting device 40 includes motor 62 coupled to the inner cannula 76 by an inner cannula drive assembly 63. The motor 62 is electrically connected to motor control unit 160 via a number, M, of signal paths 142 wherein M may be any integer. The motor control unit 160 is, in turn, connected to a number of outputs of MCU 158 via an equal number, N, of signal paths 161. Cam rotational position sensor 174 is electrically connected to a motor shaft position feedback input (SPF) of MCU 158 via signal path 162, and provides a motor stop identification signal thereon as will be more fully described hereinafter. The motor shaft stop identification signal provided by sensor 174 on signal path 162 preferably provides MCU 158 with a motor stop identification signal and may optionally provide a cutter speed signal that is proportional to the motor speed for a geared system or identical to the motor speed for a direct drive system.

Tissue cutting device 40 is further mechanically connected to a vacuum unit 168 (e.g., a combination of controllable valve 146 and vacuum generator 153 in FIG. 21A) via conduit 163, whereby the vacuum unit 168 provides a controllable vacuum level to tissue cutting device 40 for aspirating tissue received in inner cannula lumen 78. Vacuum unit 168 is electrically connected to a vacuum control unit 166 via a number, P, of signal paths 169 wherein P may be any integer. The vacuum control unit 166 is, in turn, connected to a number of outputs of MCU 158 via an equal number, L, of signal paths 167, wherein L may be any integer. A vacuum sensor 164, which may be a temperature compensated solid-state pressure sensor, may be positioned within the vacuum line 151 and electrically connected to a vacuum feedback (VF) input of MCU 158 via signal path 165. Alternatively, the vacuum sensor 164 may be disposed within hand piece 42 or within the vacuum unit 168 itself.

In operation, the MCU 158 is responsive to a vacuum command signal, preferably provided by a corresponding control mechanism associated with control panel 132, foot pedal 144, or an equivalent control mechanism, to provide one or more corresponding vacuum control signals to vacuum control unit 166 along signal paths 167. The vacuum control unit 166, in turn, is responsive to the one or more vacuum control signals to activate the vacuum unit 168 to thereby provide tissue cutting device 40 with a desired level of vacuum. The actual vacuum level provided to tissue cutting device 40 is sensed by vacuum sensor 164, which provides a corresponding vacuum feedback signal to the vacuum feedback input VF of MCU 158. The MCU 158 is then operable to compare the vacuum feedback signal with the vacuum command signal and correspondingly adjust the one or more vacuum control signals to achieve the desired vacuum level within tissue cutting device 40. Such closed-loop feedback techniques are well known in the control systems art.

In one alternative embodiment, the MCU 158 can be replaced by individual microprocessors controlling the input and output for controlling the operation of the motor 62 and the vacuum unit 168. In this alternative embodiment, the motor control and vacuum control microprocessors can be PIC16CXX Series microcontrollers provided by Microchip, Inc. of Chandler Ariz. The motor control microcontrollers can receive input signals from the motor driver circuit 172 (FIG. 23) and position sensor 174, as well as the foot switch 144 and panel controls 132. Likewise, the vacuum microcontroller can receive input signals from the vacuum sensor 164, the foot switch 144 and panel controls 138. Each microcontroller can provide its own output to its driven component and have its own display, such as an LED display, indicative of its operational status. Moreover, the two units can communicate with each other to ensure clean cutting by proper timing of the cutting and aspiration functions.

Referring now to FIG. 23, one exemplary embodiment of the motor control unit 160 is shown in greater detail. The motor control unit 160 in one embodiment includes a pulse width modulation (PWM) generator circuit 170 having a motor speed input connected to one of the MCU outputs 161₁. If motor speed control is provided, the output 161₁ can provide a variable voltage signal indicative of a desired motor speed and based upon the position of a throttle, foot pedal, or other actuator. In certain embodiments, an additional input is connected to another one of the MCU outputs 161₂. The signal at this output 161₂ can be a motor slowdown signal as described below. Alternatively, the output 161₂ can constitute a braking signal used in connection with a current feedback motor controller. As a further alternative, the slowdown command may be communicated via the motor speed command itself, rather than through a separate signal 161₂. In this instance, the output 161₂ may not be required.

In the illustrated embodiment, the PWM is disposed within the motor control unit 160. Alternatively, the PWM can be integrated into the MCU 158, or into the separate motor control microprocessor discussed above. In embodiments that include motor speed control, the motor speed input receives a motor speed signal from MCU 158 indicative of desired operational speed of the motor 62. The slowdown input can receive a speed adjustment signal from the MCU 158 based on an actual motor speed signal provided by a motor sensor associated with the motor 62.

A motor driver circuit 172 is electrically connected to PWM generator circuit 170 via signal path 173 and receives a PWM drive signal therefrom, which is a pulse width modulated signal indicative of desired motor speed. The motor driver circuit 172 provides a motor drive signal (MD) to motor 62 via signal path 175. While the disclosed embodiment contemplates digital control of the motor using the PWM generator circuit 170, alternative embodiments can utilize closed loop feedback analog circuits, particularly where slower cutting speeds are contemplated.

The motor drive signal includes a motor stop input that is connected to another one of the MCU outputs 161₁. In accordance with one aspect of the present disclosure, MCU 158 provides a motor stop signal on signal path 161₃, based on a motor deactivation command provided by foot switch 144 or panel control 138 and also based on a motor stop identification signal provided by sensor 174, to stop the inner cannula 76 in a desired position, as will be more fully described hereinafter. In certain embodiments, only the motor stop signal is utilized to command the motor to stop at the predetermined position. In these certain embodiments, the motor slowdown signal on path 161₂ can be eliminated, or the input on path 161₂ can be used for other control signals to the motor control circuit.

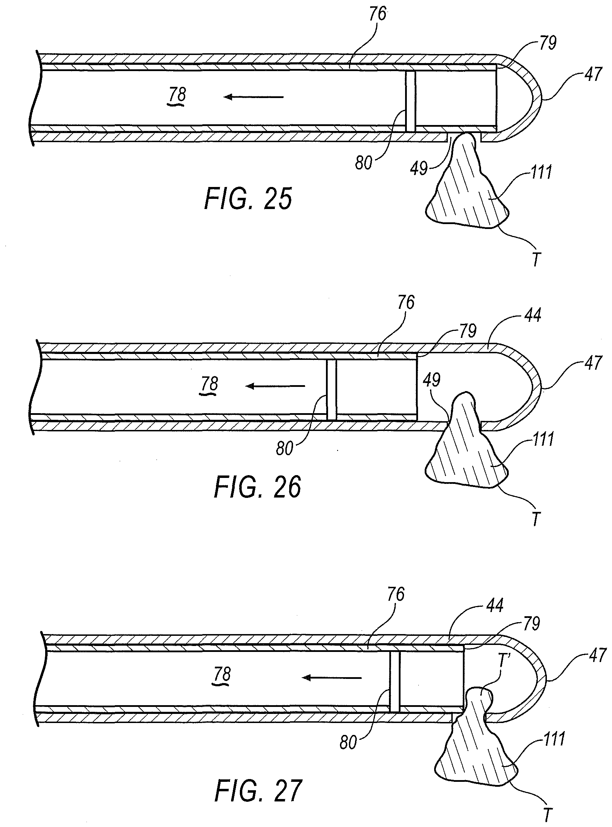

As mentioned previously, when tissue cutting device 40 is deactivated, inner cannula 76 may come to rest partially disposed within outer cannula opening 49. Referring to FIGS. 25-27, three different stop positions of inner cannula 76 are shown. FIG. 27 shows that inner cannula 76 can be stopped in a position in which a portion of the tissue T is trapped between the outer cannula opening 49 and the inner cannula distal end 79. Efforts at withdrawing outer cannula 44 from the surgical site may accordingly result in tearing of the tissue portion T' away from the surrounding tissue base T. Surgeons encountering such trapping would typically be required to re-activate tissue cutting device 40 to release the tissue portion T' from the surrounding tissue base T. To prevent such tissue trapping from occurring, deactivation of the motor 62 is controlled in such a manner that the inner cannula distal end 79 is positioned remotely from the outer cannula opening 49 when inner cannula 76 stops reciprocating. However, in certain exemplary methods of use, device 40 is used as an aspiration wand. In those methods, the stop position of inner cannula distal end 79 may be adjusted to different locations within outer cannula opening 49 in order to adjust the level of aspiration supplied to a region of the anatomy proximate outer cannula opening 49. For example, stop positions may be selected that limit the percent open area of outer cannula opening 49 to 25%, 50%, or 75% of the total area of opening 49.

Referring again to FIGS. 23 and 24, controlled deactivation of the motor 62 will now be described in detail. When it is desired to deactivate tissue cutting device 40, a motor stop command is provided such as via foot switch 144 or a panel control 138. In one embodiment, MCU 158 is responsive to the motor stop command to provide a slowdown signal to the PWM generator via signal path 161₂ which slows the action of motor 62. Preferably, the slowdown signal corresponds to a predefined signal level operable to drive the motor 62 at a motor speed below a motor speed threshold level. Since motor 62 is a brushed DC motor, it has a rotational resistance or resistive torque associated therewith as described above. In addition, in some cases friction between the inner cannula 76 and outer cannula 44 will increase the rotational resistance. Due to this combined rotational resistance, operation of the motor 62 will cease very rapidly or nearly instantly if the motor drive signal on signal path 142 is disabled while driving motor 62 below the motor speed threshold. Accordingly, when device 40 is used to cut tissue, alignment of position indicators 176a or 176b with sensor 174 preferably corresponds to a position of the tissue cutting device 40 at which there is no danger of trapping tissue between inner cannula distal end 79 and the outer cannula opening 49, and sensor 174 is operable to produce the motor stop identification signal when so aligned with indicator 176a or 176b.

In one embodiment, MCU 158 is operable to produce a motor stop signal on signal path 161₃ when sensor 174 detects alignment of position indicators 176a or 176b therewith after one passage thereby of indicator 176a or 176b since producing the slowdown signal on signal path 161₂. Allowing one passage of indicator 176a or 176b by sensor 174 after issuing the slowdown signal ensures that the rotational speed of motor 62 is at or below the motor speed threshold when subsequently issuing the motor stop command, regardless of the position of indicator 176a or 176b relative to sensor 174 when the slowdown command was issued. After one passage of indicator 176a or 176b by sensor 174 since issuing the slowdown signal, MCU 158 is responsive to the signal provided by sensor 174 indicative of alignment of indicator 176a or 176b therewith, to produce the motor stop signal on signal path 161₃. The motor driver 172 is responsive to the motor stop signal to produce a motor disable signal on signal path 175. Due to the inherent rotational resistance, motor 62 is responsive to the motor disable signal to immediately cease operation thereof with indicator 176a or 176b substantially aligned with sensor 174, and with the inner cannula 76 accordingly positioned so as not to trap tissue between inner cannula distal end 79 and the outer cannula opening 49.

As mentioned above, in one exemplary embodiment, the inner cannula stop position is user adjustable, such as by adjusting a panel control 138 on console 134. In accordance with the embodiment, it is contemplated that the stopped rotational position of cam 64, and therefore the inner cannula distal end 79, may be instead aligned with a predetermined differential distance between the indicator 176a/176b and the sensor 174. The braking characteristics of the inner cannula 76 and motor 62 can be ascertained and the stopping distance determined so that this predetermined differential distance can be calibrated accordingly. However, in a preferred embodiment, when inner cannula 76 comes to rest, the distal end 79 is located proximally of the outer cannula opening 44 by a predetermined distance, as shown in FIG. 26.

An exemplary method of using device 40 to perform a tissue cutting procedure will now be described in the context of a neurosurgical procedure involving the cutting of a neurological target tissue. In one example, the target tissue is brain tissue, and in another example the target tissue is spinal tissue, for example, the tissue of an intervertebral disk. In certain exemplary methods, the tissue specimen being cut is a tumor or a lesion.

In accordance with the exemplary method, it is first determined whether the cutting operation will be a debulking operation or a fine shaving operation or a cutting operation that is somewhere in between a debulking and fine shaving operation. A full surgical procedure may combine a variety of these procedures. A surgical access path is then created to the tissue sample of interest. In one embodiment, the surgical path is created and/or the target tissue is accessed using an "open" procedure in which the target tissue is open to the atmosphere (e.g., a full open craniotomy). In another example, the surgical path is created and/or the target tissue is accessed using a "closed" procedure in which the target tissue is sealed from the atmosphere.

At this point, the distal end 79 of inner cannula 76 is located proximally of outer cannula opening 49 due to the use of an inner cannula stop position control of the type described previously. The maximum vacuum level to be applied to device 40 is then set using panel controls 138. Generally, higher vacuum levels will be used for debulking procedures than for fine shaving procedures as higher vacuum levels will tend to draw relatively larger sections of tissue into outer cannula opening 49. In one embodiment, the panel control 138 is a knob on console 134 that is rotated to set the desired maximum vacuum level.

In one arrangement, device 40 is configured to be gripped with a single hand during a tissue cutting procedure. A variety of different grips may be used, examples of which are provided in FIGS. 29A-29C. Referring to FIG. 29A, in one example, device 40 is gripped by wrapping the four fingers of one hand around the proximal end 46 of lower housing 50 with device 40 resting in the palm of the user's hand. In this configuration, the pinky finger is spaced apart from the proximal end of lower housing 50 and is located adjacent the midpoint MP of the lower housing 50. The thumb is spaced apart from midpoint MP and is adjacent the proximal end of lower housing 50. In FIG. 29A, the thumb is located adjacently distal to hose connector 43. In the configuration of FIG. 29A, the right hand is used to grip device 40, and when viewed from the proximal end of lower housing 50, the fingers of the hand wrap around the lower housing 50 in a counterclockwise direction. If the left hand were used, the fingers would wrap around lower housing 50 in the opposite (e.g., clockwise) direction.

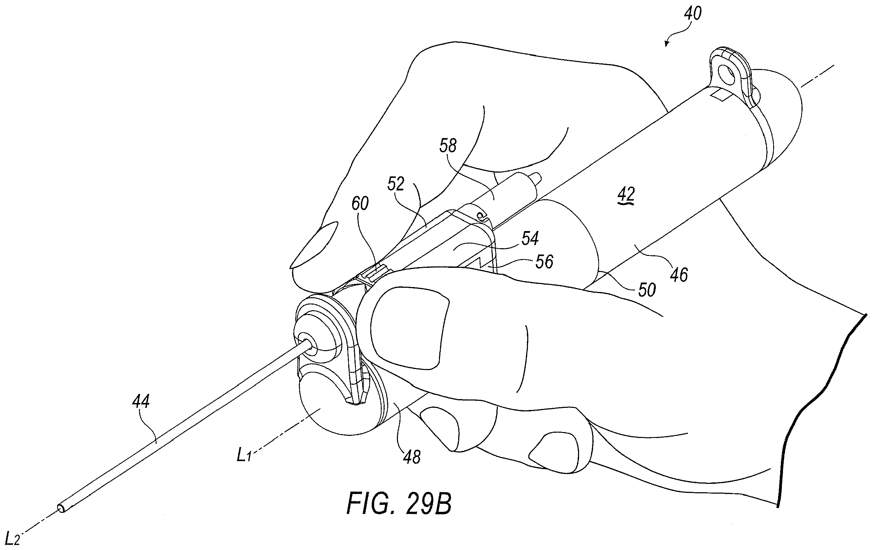

Referring to FIG. 29B, another single-hand gripping orientation is depicted. The depicted orientation is similar to the grip that is used to hold a writing instrument, such as a pen or pencil. The index finger engages a portion of the upper housing 52 and the thumb engages a portion of the upper housing 52 and/or a portion of lower housing 50. The index finger and thumb are preferably spaced apart in a direction that is perpendicular to upper housing longitudinal axis L₂. Proximal portion 46 of lower housing 50 rests between the base of the index finger and the base of the thumb. In one preferred example, the grip of FIG. 29B is used when the center of gravity of device 40 is distal of lower housing midpoint MP. The grip of FIG. 29B allows the device 40 to be used for delicate shaving and debulking procedures in which relatively precise manipulation of device 40 is desirable. In addition, when held in the grip of FIG. 29B, rotation dial 60 can be readily rotated with the index finger to adjust the circumferential orientation of the cannulae 44 and 76 without using a second hand or repositioning the fingers of the gripping hand.

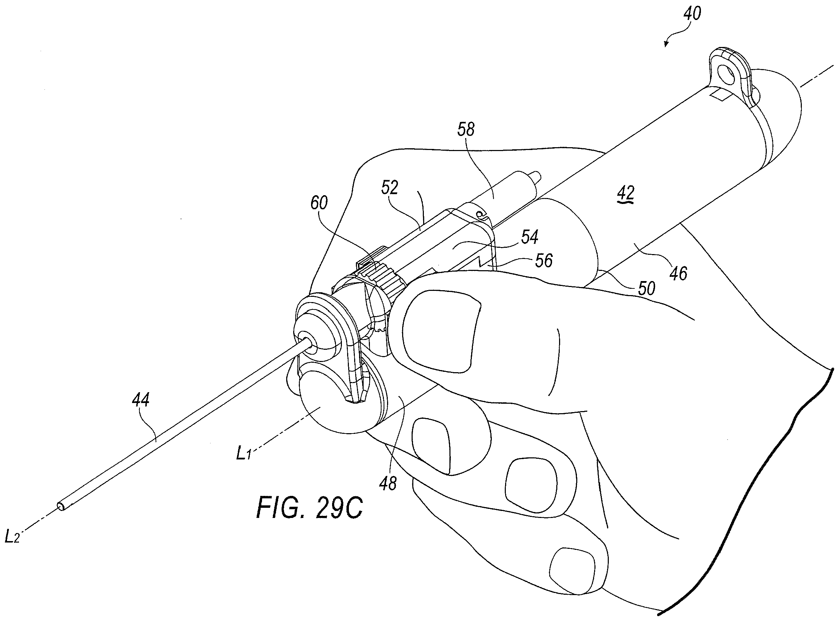

Referring to FIG. 29C, a further single-hand gripping orientation is depicted. In this orientation, the thumb is positioned generally as described and depicted with respect to FIG. 29B. However, the tip of the index finger engages the lower housing 50 instead of upper housing 52. The index finger and thumb are again spaced apart from one another in a direction that is perpendicular to the longitudinal axis L₂ of upper housing 52, and the proximal housing section 46 rests between the base of the thumb and index finger. For additional support, the medial side of the middle finger is also placed against the lower housing 50.

After grasping handpiece 42, the surgeon may use the proximal end of upper housing 52 to align the outer cannula 44 with the target tissue. Referring to FIG. 28, the surgeon may view the proximal end of upper housing 52 and use it as a "gun sight" to perform the alignment. The surgeon will then insert outer cannula 44 to a location proximate the target tissue. Depending on the hand and the surgeon's orientation with respect to the target tissue, the surgeon may then rotate dial 60 to rotate outer cannula 44 about its own longitudinal axis L₂ and orient outer cannula opening 49 immediately adjacent the target tissue. The rotation of outer cannula 44 with dial 60 causes inner cannula 76 to rotate such that a fixed rotational or angular relationship is maintained between inner cannula 76 and outer cannula 44. Once the opening 49 is in the desired orientation, the motor 62 is activated, for example, by beginning to depress pedal 144 from its fully undepressed (open) position to a second partially depressed position which causes motor control unit 160 to send a signal to motor 62 on signal path 142. Motor 62 may also be activated by a panel control 138. The rotation of motor 62 causes cam 64 to rotate, resulting in the reciprocation of cam follower 68 and cam transfer 72. The reciprocation of cam transfer 72 causes cannula transfer 74 to reciprocate, thereby reciprocating inner cannula 76 within outer cannula lumen 110.

Once motor 62 is activated, vacuum is supplied to inner cannula lumen 78. In one embodiment, as the pedal 144 is further depressed (beyond the position at which motor 62 is activated), vacuum generator 153 is activated. The surgeon then adjusts the degree of depression of the foot pedal 144 to obtain the desired level of vacuum by visualizing the movement of the target tissue relative to the outer cannula opening 49. In certain embodiments, the surgeon controls the vacuum level to obtain a desired amount of traction in the tissue surrounding the target tissue. If the surgeon desires to apply the previously set maximum vacuum level, he or she depresses pedal 144 to its fully depressed position.

If desired, the surgeon may depress and partially release the pedal 144 a number of times to manipulate the target tissue in a satisfactory manner. Open loop or closed loop vacuum control may be provided. In one example, closed loop control is used. In accordance with the example, vacuum control unit 166 is manipulable to adjust the setpoint of vacuum generator 153 which is manipulable to adjust the inner cannula vacuum level along a continuum of levels below the pre-selected maximum level. In one embodiment, the extent of depression of foot pedal 144 dictates the vacuum set point supplied to vacuum control unit 166 on signal path 167, and therefore, the amount of vacuum provided by vacuum unit 168. Vacuum sensor 164 measures the vacuum supplied to tissue collector 58 and feeds a signal back to main control unit 158 on signal path 165. The measured vacuum is then compared to the set point applied to vacuum control unit 166 via foot pedal 144, and the signal transmitted to vacuum generator 153 is then adjusted to move the measured vacuum value towards the set point. To obtain a vacuum level equal to the maximum pre-set level, pedal 144 is completely depressed.

In another example, the vacuum system is operated in an open loop manner. In accordance with the example, pedal 144 directly adjusts vacuum unit 166. In one implementation, pedal 144 variably adjusts the amount of pressure supplied to controllable valve 146. The variation in pressure in turn affects the vacuum provided by vacuum generator 153 to inner cannula lumen 78. As with the closed loop example, the system may be configured to allow the user to set a maximum vacuum that is obtained when pedal 144 is fully depressed.

Maximum vacuum levels of at least about 0 in Hg. are preferred, and maximum vacuum levels of at least about 1 in Hg. are more preferred. Maximum vacuum levels of at least about 5 in Hg. are even more preferred, and maximum vacuum levels of at least about 10 in Hg. are still more preferred. Maximum vacuum levels of at least about 20 in. Hg. are yet more preferred, and vacuum levels of at least about 29 in. Hg. are most preferred.

Due to the resistance of the tissue drawn into outer cannula opening 49, cutting section 83 pivots about hinge 80 and toward outer cannula opening 49 as inner cannula 76 travels in the distal direction. The inner cannula cutting section 83 continues to pivot as it travels in the distal direction, eventually compressing tissue within outer cannula opening 49 and severing it. The severed tissue forms a continuum of tissue snippets 112 (FIG. 14) within inner cannula lumen 78. Due to the vacuum applied to tissue collector 58, snippets 112 are aspirated through inner cannula lumen 78 in the proximal direction. They eventually exit inner cannula lumen 78 at inner cannula proximal end 77 and enter tissue collector 58 (or fluid collection canister 192 if no collector 58 is provided). Any fluids that are aspirated exit tissue collector 58 and are trapped in fluid collection canister 192. The surgeon preferably severs tissue at a cutting rate of at least about 1,000 cuts/minute. Cutting rates of at least about 1,200 cuts/minute are more preferred, and cutting rates of at least about 1,500 cuts/minute are even more preferred. Cutting rates of less than about 2,500 cuts/minute are preferred. Cutting rates of less than about 2,000 cuts/minute are more preferred, and cutting rates of less than about 1,800 cuts/minute are even more preferred.

The surgeon may move device 40 around the target tissue until the desired degree of cutting has been completed. Motor 62 is then deactivated, for example, by completely releasing pedal 144 so it returns to its fully undepressed (open) position. If an inner cannula stop position control is provided, inner cannula 76 preferably comes to rest proximally of outer cannula opening 49, as shown in FIG. 26. Outer cannula 44 is then removed from the surgical site. Tissue collector 58 is then removed from upper housing 52 of handpiece 42, and the collected tissue samples are either discarded or saved for subsequent analysis. Fluids collected in canister 192 are preferably discarded. If the remote tissue collector of FIG. 21A is used, tissue samples may be removed from it without removing outer cannula 44 from the surgical site or otherwise disturbing the surrounding tissue.

In certain exemplary methods, tissue cutting device 40 may be used with an imaging device, for example, a video camera or a magnification instrument such as a microscope or an endoscope. The imaging device aids the surgeon in visualizing the target tissue to be resected and allows for more precise surgical techniques. Tissue cutting device 40 is advantageously grippable and manipulable with one hand, allowing the surgeon to manipulate an imaging device with his or her other hand. As mentioned previously, the offsetting of the cannulae 44 and 76 from the lower housing 50 allows the device to be shortened relative to other devices. Thus, device 40 is more readily manipulable in restricted spaces that are sometimes encountered in imaging device-assisted neurosurgical procedures.

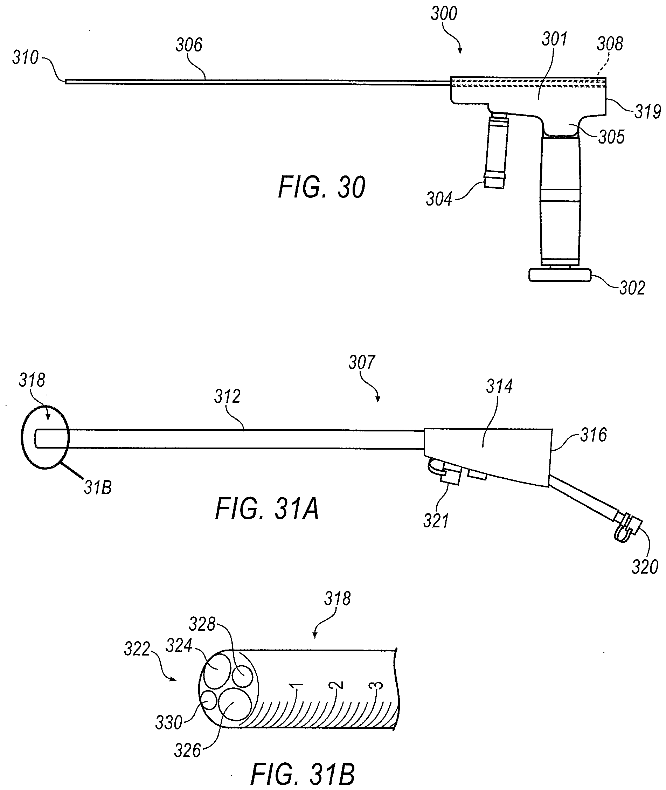

Referring to FIG. 30, an endoscope 300 for use with tissue cutting device 40 is depicted. Endoscope 300 comprises a housing 301, an eye-piece 302, a fiber optic cable connector 304, and a shaft 306. Shaft 306 includes a proximal end 308 which is disposed in and connected to housing 301. Shaft 306 further includes a distal end 310 spaced apart from proximal end 308. Endoscope 300 is configured to allow a user to view a surgical area of interest proximate distal shaft end 310 through eye-piece 302. Shaft 306 includes a conduit (not separately shown) for transmitting light provided via fiber optic connector 304 to the surgical area. Shaft 306 also includes a lens (not separately shown) for magnifying and viewing the surgical area. Eye-piece 302 is pivotally connected to housing 301 at pivot axis 305, allowing eyepiece 302 to be adjusted to various positions about pivot axis 305. As a result, eye-piece 302 can be moved toward distal shaft end 310 or away from distal shaft end 310. Eye-piece 302 may also be connected to a camera with a camera connector so that the image generated by endoscope 300 can be viewed on a display monitor.

To facilitate the use of endoscope 300 in surgical procedure, trocar 307 may be provided, as best seen in FIGS. 31A and 31B. Trocar 307 is especially useful for closed surgical procedures. Trocar 307 comprises a trocar body 314 and a trocar shaft 312. Trocar 307 has a proximal end 316 with a proximal opening formed in trocar body 314 and a distal end 318 on shaft 312. Shaft 312 defines one or more channels in its interior. Trocar body 314 is sized to accommodate irrigation conduit 320 as well as endoscope 300 and tissue cutting device 40. As shown in FIG. 31B, shaft 312 has a plurality of channels, 324, 326, 328, and 330 which terminate at tip distal face 322. Working channel 324 is sized to accommodate outer cannula 44 of tissue cutting device 40. Channel 326 is sized to accommodate endoscope shaft 306. Channel 328 is an irrigation channel used to direct irrigation fluid from irrigation conduit 320 to the surgical site. Channel 330 is a relief channel used to relieve fluid pressure at the surgical site. During closed procedures, as irrigation fluid flows to a surgical site it can pressurize the site. If left unchecked, such pressurization can result in tissue and/or neurological damage. Thus, relief channel 330 provides a fluid path to relieve pressure build up at the surgical site.

In certain examples, tissue cutting device 40 is combined with an imaging device to define a tissue cutting and imaging assembly 303 that is capable of simultaneously imaging and cutting a target tissue associated with a patient's neurological system. Because assembly 303 effectively combines both imaging and cutting operations into a single, integral device, it is particularly advantageous in performing closed procedures where a surgical access path is created percutaneously.

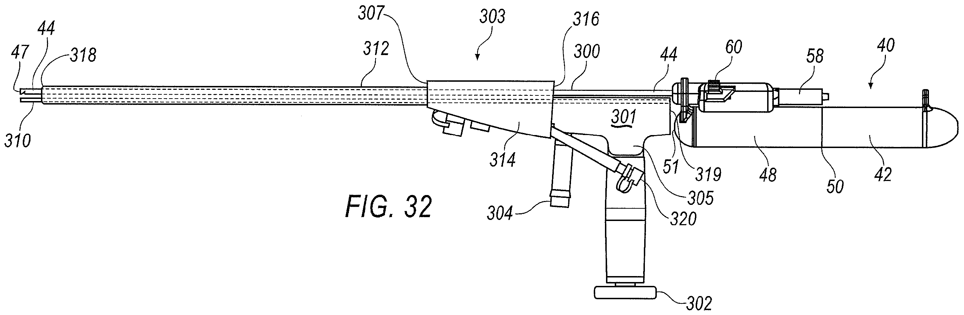

Referring to FIG. 32, a tissue cutting and imaging assembly 303 is depicted. Cutting and imaging assembly 303 comprises tissue cutting device 40, trocar 307, and endoscope 300. As shown in the figure, endoscope 300 is inserted through trocar 307 via endoscope channel 326 such that endoscope distal end 310 exits through and projects away from trocar 307 at trocar shaft distal tip face 322. Tissue cutting device 40 is connected to trocar 307 such that outer cannula 44 is inserted in the open proximal end 316, through trocar body 314, and through working channel 324 of trocar shaft 312. Distal end 47 of the outer cannula 44 of tissue cutting device 40 projects through and away from shaft distal end 318 of trocar 307 at trocar shaft distal tip face 322.

Although various configurations are possible, in the cutting and imaging system 303 of FIG. 32, tissue cutting device 40 is positioned with a proximal portion of outer cannula 44 adjacent to endoscope housing 301. Proximal end 319 of endoscope housing 301 is positioned distally of and adjacent to front housing 55 of tissue cutting device handpiece 42.

An exemplary method of using the cutting and imaging assembly 303 of FIG. 32 to perform a tissue cutting procedure will now be described in the context of a neurosurgical procedure involving the cutting of a neurological target tissue. In one example, the target tissue is a brain tissue. In another example, the target tissue is spinal tissue, for example, the tissue of an intervertebral disk. In certain exemplary methods, the tissue specimen being cut is a tumor or lesion.

As with the previous method of use discussed above, it is first determined whether the cutting operation will be a debulking operation, a fine shaving operation, or an operation that is somewhere in between a debulking operation and a fine shaving operation. In addition, any given surgical procedure may combine various debulking and fine shaving operations. Based on the nature of the cutting operation, the user may set a maximum vacuum level in the manner described previously.

Tissue cutting and imaging assembly 303 is provided in the form in which it appears in FIG. 32. A surgical access path is created and/or the target tissue is accessed using an open procedure or a closed procedure, as explained previously. However, tissue cutting and imaging assembly 303 is especially suited for closed procedures. In one example, the surgeon places one eye at eye-piece 302 and manipulates trocar shaft 312 to position distal trocar tip 318 proximate the target tissue. At this point, distal end 79 of inner cannula 76 is located proximally of outer cannula opening 49 due to the use of inner cannula stop position control of the type described previously. The maximum vacuum level to be applied to device 40 is then set using panel controls 138. As mentioned previously, higher vacuum levels will generally be used for debulking procedures while relatively lower vacuum levels will be used for fine shaving procedures. In one exemplary method, device 40 is configured to be gripped with a single hand so as to allow simultaneous manipulation of endoscope 300, trocar 307, and tissue cutting device 40. A variety of different grips may be used, as discussed previously with respect to FIGS. 29A-29C.

Depending on the selected hand and the surgeon's position with respect to that of the target tissue, dial 60 may be rotated to rotate outer cannula 44 about its own longitudinal axis and to orient outer cannula opening 49 immediately adjacent the target tissue. As discussed previously, the tissue removal device 40 is preferably configured such that when outer cannula 44 rotates, inner cannula 76 also rotates to maintain a fixed angular orientation between outer cannula 44 and inner cannula 76. Once the opening is in the desired position, motor 62 is activated in the manner described previously. Vacuum generator 153 is then activated and foot pedal 144 is used to obtain the desired level of vacuum. In one example, the surgeon views the target tissue through eyepiece 302 to visualize the tissue's response (e.g., traction) to various levels of vacuum and select a desired level. The vacuum levels are the same as those described previously.

Due to the application of vacuum, the target tissue proximate trocar distal end 318 is drawn into outer cannula opening 49. As inner cannula 76 travels in the distal direction, it compresses tissue received within outer cannula opening 49, causing inner cannula 76 to pivot about hinge 80 and sever the received tissue in to discrete tissue samples such as tissue snippets. As described earlier with reference to FIG. 14, tissue snippets 112 are aspirated through inner cannula lumen 78 in the proximal direction due to the application of vacuum. Snippets 112 eventually enter tissue collector 58 (or fluid collection canister 192 if a remote collection system such as that of FIG. 21A is used). The tissue cutting rates are preferably those described previously. If desired, an irrigation fluid such as saline may be fed to the target tissue area via irrigation conduit 320.

Endoscope 300 is configured to allow a surgeon to view the target tissue through eye-piece 302. However, a camera may also be connected to a camera connector (not shown) attached to eyepiece 302 allowing the image generated by endoscope 300 to be viewed on a display monitor. In accordance with one example, the surgeon views the target tissue on the display monitor while manipulating cutting and imaging system 303 and cutting tissue.

In certain examples, device 40 is configured such that its outer cannula 44 can be accommodated by working channels in known trocars. In certain embodiments, working channel 324 has an inner diameter of less than 8mm, preferably less than 6mm, more preferably less than 4mm, and most preferably about 2mm, and outer cannula 44 has an outer diameter that allows outer cannula 44 to be slidably received in working channel 324. In other examples, outer cannula 44 is at least as long as known working channels. In certain embodiments, outer cannula 44 is at least about 6 inches, preferably at least about 8 inches, more preferably at least about 10 inches, and even more preferably at least about 12 inches in length. Unlike many known neurosurgical devices such as rotrary shavers or ultrasonic devices, in certain exemplary implementations, owing to the rate of reciprocation and the inclusion of hinge 80, tissue cutting device 40 is capable of cutting tissue samples having at least one dimension that is smaller than the inner diameter of inner cannula 76 while having an outer cannula 44 diameter that is small enough to fit into the working channels of known trocars.

Tissue cutting and imaging assembly 303 is useful in a number of procedures, but is especially beneficial in closed procedures. In one exemplary method, tissue cutting and imaging assembly 303 is used to perform closed, percutaneous tissue cutting procedures in the third ventricle of the brain. Such procedures include removing tumors and membranes in the third ventricle. In addition, cerebrospinal fluid circulates through the third ventricle and into the spinal column. In certain patients, occlusions can form in the third ventricle, blocking the fluid circulation. Tissue cutting and imaging assembly 303 may be used to remove such occlusions and restore circulation. Other closed procedures for which tissue cutting and imaging assembly is particularly well suited include the removal of tumors from the hypothalamus.

As mentioned previously, tissue cutting device 40 may be operated as a variable aspiration wand. This mode of operation may be particularly useful if tissue cutting and imaging device 303 is provided. In one exemplary method, tissue cutting device 40 is operated in both a tissue cutting mode and in an aspiration wand mode (i.e., the inner cannula 76 is not reciprocating) without being removed from working channel 324 between modes. In addition, the inner cannula stop position may be adjusted to obtain a desired degree of aspiration or vary the degree of aspiration, and therefore, the vacuum level applied to inner cannula lumen 78. This avoids a difficulty present in certain existing methods wherein a cutting device must be removed from the working channel so that a separate aspiration wand may be inserted in it.

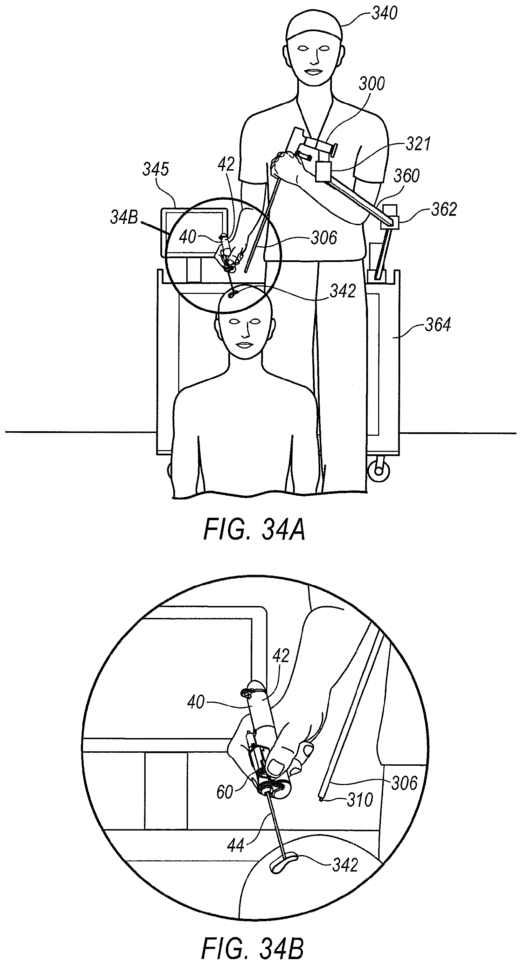

In accordance with another exemplary method, endoscope 300 and tissue cutting device 40 may be independently manipulated by a surgeon without being combined into an integral cutting and imaging assembly 303. Referring to FIG. 34A, surgeon 340 grips tissue cutting device handpiece 42 in one hand while holding endoscope 300 (or holding trocar 307 with an inserted endoscope 300) in the other hand. A variety of grips may be used to hold device 40 as described above. An articulating arm 360 may be connected to a heavy or relatively stationary object such as cabinet 364. Articulating arm 360 is connected to trocar 307 via connector 321 which may be any known type of connector. As shown in FIG. 34B, distal end 310 of endoscope shaft 306 is positioned proximate target tissue 342 to allow it to be visualized by endoscope 300 (and, optionally, on monitor 345 if a camera connection is provided). The distal end 47 of tissue cutting device outer cannula 44 is positioned in a line of sight defined between distal endoscope end 310 and target tissue 342 to allow the target tissue to be visualized as it is manipulated and/or cut by tissue cutting device 40. In one exemplary method, endoscope 300 is positioned such that the entire length of outer cannula 44 is disposed between endoscope eyepiece 302 and target tissue 342 in a direction along the longitudinal axis of endoscope 300. Once tissue cutting device 40 is positioned as desired, the reciprocation of the inner cannula 76 can be initiated, and vacuum can be applied to cut target tissue 342 in the manner described previously. If desired, endoscope 300 can be used to view target tissue 342 before and/or during reciprocation of inner cannula 76. Tissue cutting device 40 may be operated in a tissue cutting mode and in an aspiration wand mode without removing outer cannula 44 from the field of view defined between distal endoscope tip 310 and target tissue 342.

As mentioned previously, an irrigation fluid may be supplied to the surgical site via irrigation channel 328 in trocar 307. If the pressure at the surgical site becomes too great, relief channel 330 will relieve at least some of the excess fluid to drop the pressure. In addition, device 40 may provide additional pressure relief. As mentioned previously, in certain exemplary embodiments wherein device 40 is operated at reciprocation rates of 1500 reciprocations/minute or more, the combination of the reciprocation rate and the inclusion of a hinged inner cannula produces discrete tissue samples having at least one dimension that is less than the inner diameter of inner cannula 76. In such cases, there will be a fluid flow path around the severed tissue samples as they are aspirated through inner cannula 76. This fluid flow path provides an additional mechanism for relieving excess pressure at the surgical site, which can be helpful if relief channel 330 is at capacity or becomes occluded.

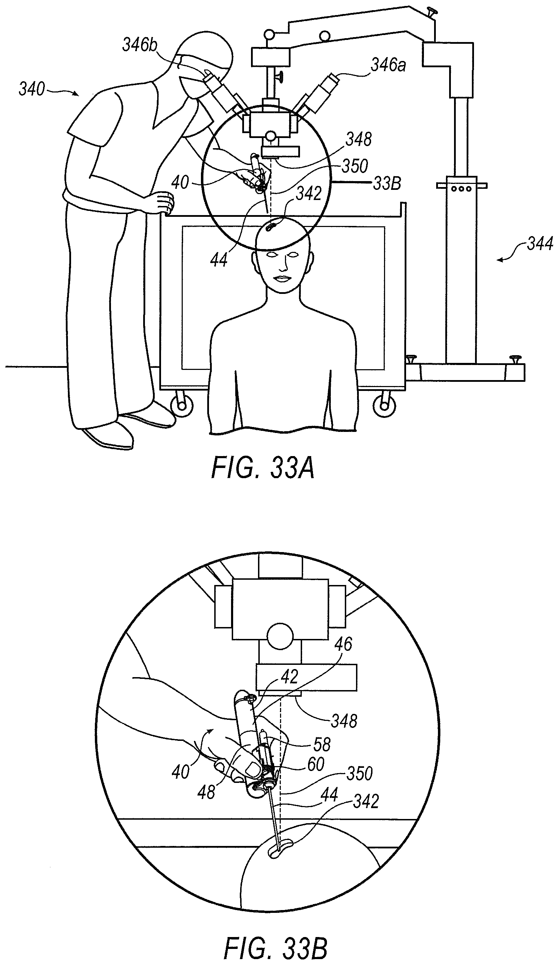

As discussed earlier, tissue cutting device 40 may also be used with microscopes during tissue cutting and removal procedures. One such procedure is an open craniotomy, an example of which is depicted in FIGS. 33A-33B. A surgical opening to target tissue 342 is created in the patient's cranium to provide access to the target tissue to be removed. In order to better visualize the target tissue, microscope 344 is provided. A variety of different microscope designs and configurations may be used, and the microscope depicted in FIG. 33A is merely exemplary. Microscope 344 includes eyepieces 346a and 346b through which surgeon 340 visualizes the target tissue. Lens 348 is provided to magnify the target tissue image provided to eyepieces 346a and 346b. Microscope 344 is positioned such that a line of sight 350 is defined between lens 348 and target tissue 342. While viewing the target tissue through one of the eyepieces 346a, 346b, surgeon 340 grips tissue cutting device 40 in one hand and positions the distal end 47 of outer cannula 44 such that outer cannula opening 49 is proximate the target tissue 342. The variety of grips described earlier may be used to hold device 40. If necessary, rotation dial 60 is used to adjust the circumferential position of outer cannula opening 49 with respect to target tissue 342. In certain exemplary embodiments, outer cannula 44 has a length that is less than the distance between lens 348 and target tissue 342, allowing surgeon 340 to better manipulate tissue cutting device 40 without inadvertently contacting lens 348. In other embodiments, tissue cutting device 40 has a length that is less than the distance between lens 348 and target tissue 342.

Once device 40 is positioned as desired relative to target tissue 342, device 40 is activated in the manner described previously to cause the generation of vacuum in inner cannula lumen 78 and the reciprocation of inner cannula 76 within outer cannula 44. Target tissue 342 is then received in outer cannula opening 49 and severed by the cutting edge of inner cannula distal end 79. Severed tissue samples are then aspirated and collected by tissue collector 58 (which may be attached to device 40 or positioned remotely from it). In certain examples, target tissue 342 is viewed with microscope 344 before inner cannula 76 begins reciprocating within outer cannula 44. In other examples, target tissue 342 is first viewed prior to reciprocation and then viewed again during reciprocation. As discussed previously, in certain implementations, tissue cutting device 40 is capable of cutting small snippets of tissue. Unlike certain known devices, in certain examples device 40 is particularly well suited for use with imaging devices such as microscopes and endoscopes because the size of the severed tissue samples is more commensurate with the level of magnification provided. Thus, tissue cutting may occur on a smaller scale and can be viewed at higher magnifications. Device 40 may provide a synergistic effect when used with imaging devices because it is manipulable in smaller spaces typically encountered in some imaging device-assisted neurological procedures and because it allows for more refined and smaller scale tissue cutting. In those exemplary implementations wherein device 40 is operable as a variable aspiration wand, it may be operated both in a tissue cutting mode and in an aspiration mode without being removed from the field of view of the imaging device.

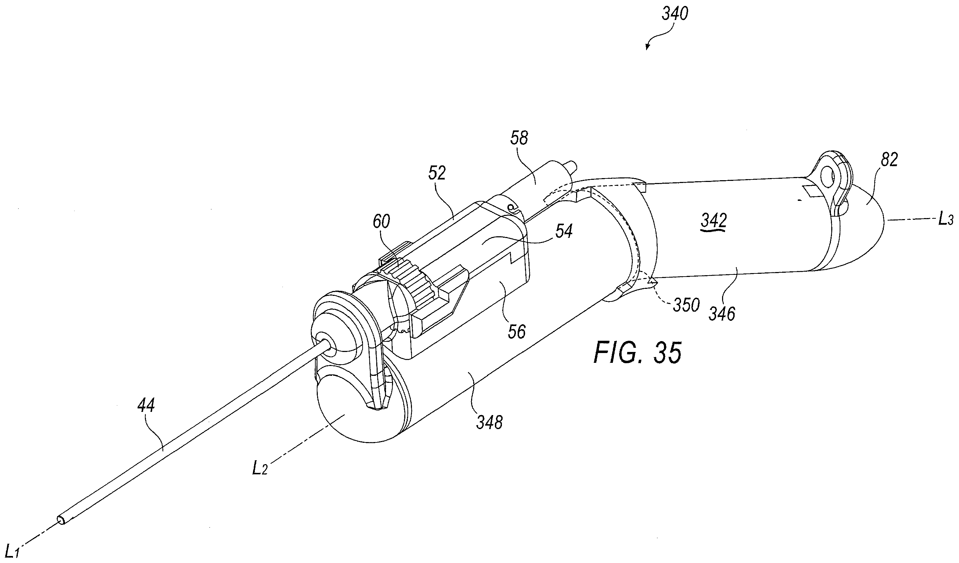

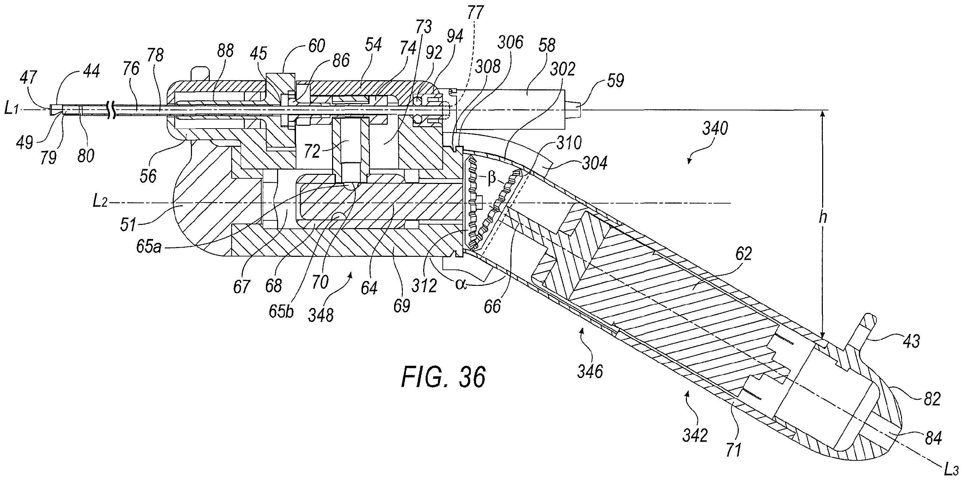

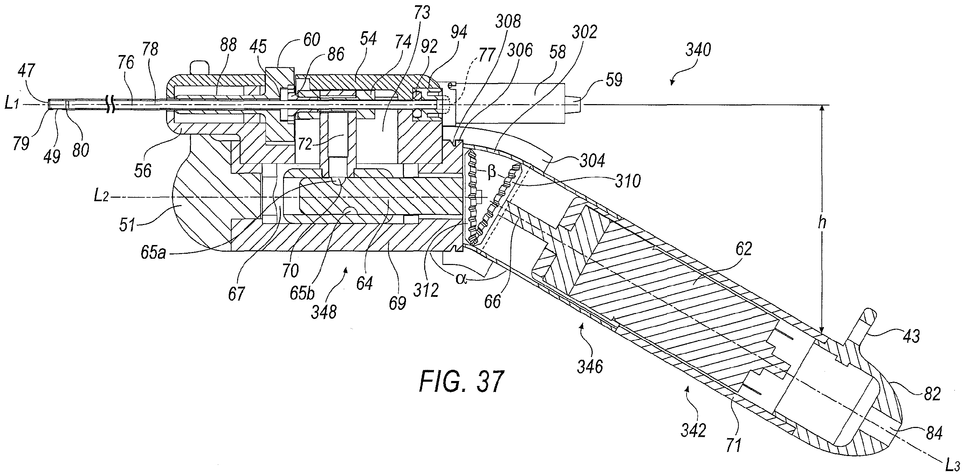

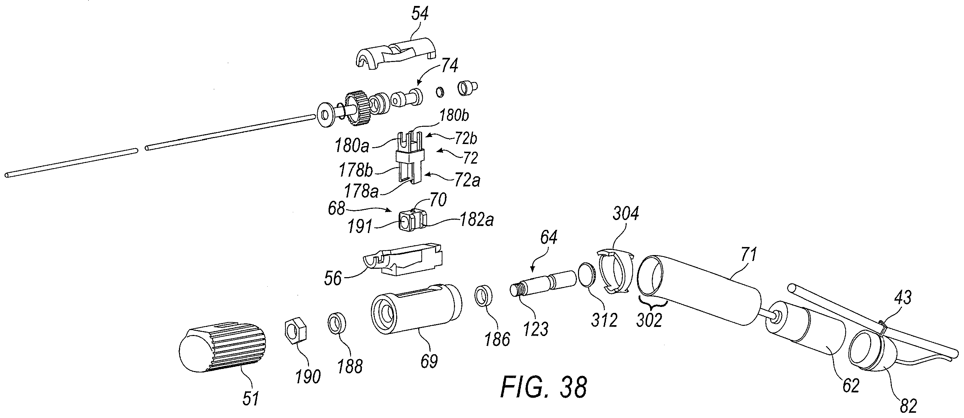

Referring to FIG. 35 an alternative embodiment of a tissue cutting device 340 is depicted. Tissue cutting device 340 is configured similarly to and operates in a similar manner to tissue cutting device 40. However, tissue cutting device 340 includes an angled handpiece 342 as well as some modified components which facilitate the use of an angled handpiece. For ease of reference, those components that are the same as those in the embodiment of FIG. 1 are identified by the same reference numerals used previously. Components that are new or which have been modified relative to the embodiment of FIG. 1 have been assigned a "300" series figure number. FIGS. 36 and 37 depict device 340 with inner cannula 76 in a proximal position and a distal position, respectively. FIG. 38 depicts an exploded view of device 340.

In accordance with the embodiment, tissue cutting device 340 includes a handpiece 342 and an outer cannula 44 that is offset from the handpiece 342. Unlike the previous embodiments of a tissue cutting device, handpiece 342 is angled, not straight. Thus, handpiece 342 includes a lower housing 350 which comprises a proximal handpiece section 346 and distal handpiece section 348. Lower housing 350 comprises a proximal-most housing portion 82 (FIGS. 2 and 3) that is connected to a motor housing 71, and a cam housing 69 that is connected to motor housing 71. A front housing section 55 is connected to cam housing 69. Upper housing 52 is also provided and is connected to lower housing distal handpiece section 348. Upper housing 52 is spaced apart from cam 64 in the radial direction of distal handpiece section 348, i.e., in a direction that is perpendicular to inner cannula longitudinal axis L₁. A tissue collector 58 may be remotely or directly connected to upper housing 52 as described previously. A rotation dial 60 for rotating the outer cannula 44 with respect to handpiece 42 is also mounted to upper housing 52.

Inner cannula 76 is disposed in outer cannula 44 and reciprocates within outer cannula lumen 110 in the manner described previously. Inner cannula 76 has a longitudinal axis L₁ extending along its length through its radial center point. Inner cannula 76 and outer cannula 44 are partially disposed in upper housing 52 and project away from its distal end.

Distal handpiece section 348 has a longitudinal axis L₂ extending along its length through the radial center point of cam 64. Inner cannula longitudinal axis L₁ is spaced apart from and substantially parallel to distal handpiece section longitudinal axis L₂. Proximal handpiece section 346 and distal handpiece section 348 define an angle α (FIGS. 36-37) between proximal handpiece section 346 and distal handpiece section 348. In certain examples, the angled handpiece 342 allows the surgeon to grip tissue cutting device 340 at an orientation that is angled with respect to the direction of surgical entry into the patient. In certain other examples, distal handpiece section 348 is grippable with a single hand, and the angled design provides a clearance between proximal handpiece section 346 and adjacent instruments or devices.

The angle α between distal handpiece section 348 and proximal handpiece section 346 is preferably set to provide a desired degree of manipulability of device 340 while preventing the device 340 from contacting adjacent instruments or devices. The angle α preferably ranges from at least about 90° to less than 180°, more preferably from about 110° to about 160°, even more preferably from at least about 130° to about 150°, still more preferably from at least about 140° to about 160°, and most preferably from about 145° to about 155°.

Proximal handpiece section 346 has a longitudinal axis L₃ that extends through its radial centerpoint. As indicated in FIG. 36, axis L₃ intersects both axis L₂ and axis L₃. Proximal handpiece section 346 is spaced apart from inner cannula longitudinal axis L₁ in a direction h that is substantially perpendicular to inner cannula longitudinal axis L₁. In addition, the spacing between inner cannula longitudinal axis L₁ and proximal handpiece section 346 (i.e., the spacing in direction h) increases from the distal end of proximal handpiece section 346 to the proximal end of proximal handpiece section 346.

Distal end 302 (FIGS. 36-37) of proximal handpiece section 346 is preferably curved to facilitate its attachment to the proximal end of distal handpiece section 348. In one example, proximal handpiece section 346 is removably attachable to distal handpiece section 348. In another example, proximal handpiece section 346 and distal handpiece section 348 are configured for a snap-fit connection to one another. One such snap-fit design is illustrated in FIGS. 36, 37, and 38. In accordance with the figures, handpiece 342 includes a collar 304 that is fixedly attached to the curved distal end 302 of proximal handpiece section 346. Collar 304 includes a distal, radially inward projecting ridge 306. Ridge 306 is preferably constructed of a resilient material such as a resilient plastic. The proximal end of distal handpiece section 348 includes a groove or channel 308 into which ridge 306 fits. Thus, collar 304 can be slid over the proximal end of distal handpiece section 348 allowing the ridge 306 to snap into place in groove 308. Connection mechanisms other than ridges and grooves may be used. For example, collar 304 may include a plurality of radially inward facing tabs that engage complementary slots formed in the proximal end of distal handpiece section 348. Proximal handpiece section 346 may be connected to distal handpiece section 348 by other or additional means such as an adhesive or mechanical fasteners as well.

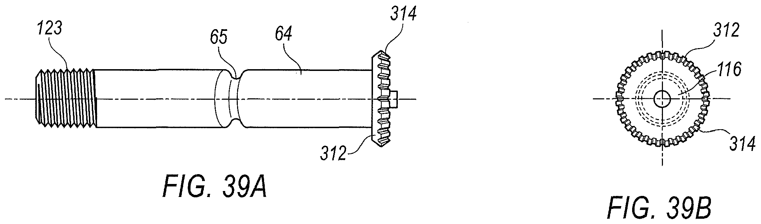

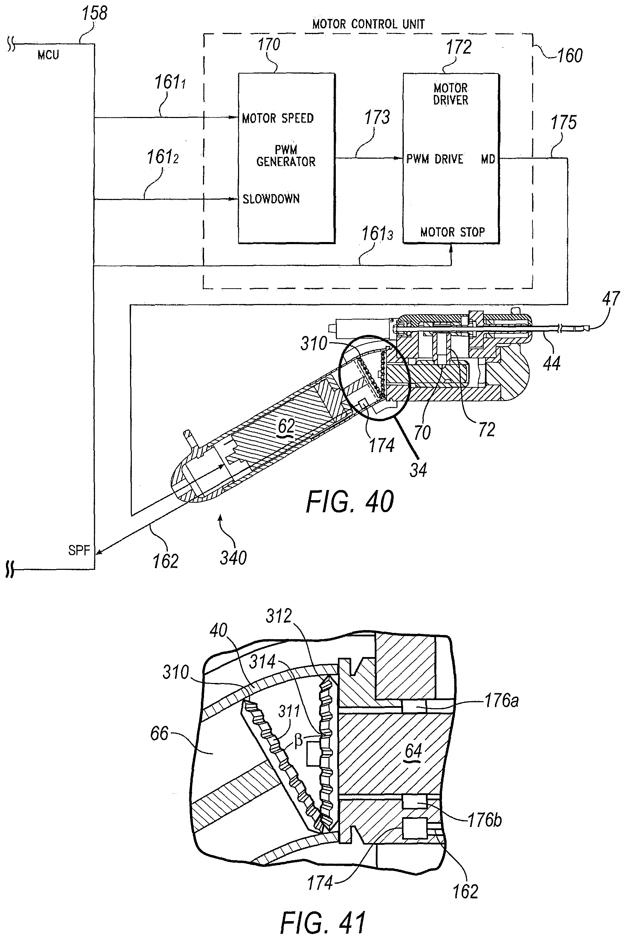

As with the previous embodiment, motor 62 is operatively connected to cam 64 such that when motor shaft 66 rotates about proximal handpiece section longitudinal axis L₃, cam 64 rotates about distal handpiece section longitudinal axis L₂. However, in the embodiment of FIGS. 35-41, motor 62 rotates about an axis (L₃) that is not collinear with the axis (L₂) about which cam 64 rotates. A rotary motion conversion mechanism is preferably provided to convert the rotation of motor 62 about axis L₃ to the rotation of cam 64 about axis L₂. One such mechanism is depicted in FIGS. 36-37. As shown therein, motor 62 includes a shaft 66 with a drive gear 310 mounted to the distal end of shaft 66. Drive gear 310 engages a driven gear 312 that is mounted on the proximal end of cam 64, as best seen in FIGS. 39A and 39B. In one example, drive gear 310 has teeth 311 (FIG. 41) that intermesh with complementary teeth 314 formed on driven gear 312. In the implementation depicted in FIGS. 36-41, both drive gear 310 and driven gear 312 are beveled gears that engage one another to define an angle β between drive gear 310 and driven gear 312. The angle β preferably ranges from greater than 0° to about 90°, more preferably ranges from about 20° to about 70°, even more preferably ranges from about 30° to about 60°, and still more preferably ranges from about 40° to about 50°.

Tissue cutting device 340 operates identically to device 40 in all respects except for the manner in which the rotation of motor 62 is converted to the rotation of cam 64. Thus, when motor 62 is energized, it rotates, causing shaft 66 and drive gear 310 to rotate about proximal handpiece section longitudinal axis L₃. The engagement of drive gear 310 and driven gear 312 causes driven gear 312 to rotate about distal handpiece section longitudinal axis L₂. Because driven gear 312 is mounted on cam 64, as driven gear 312 rotates, cam 64 begins to rotate about distal handpiece section longitudinal axis L₂. The ball bearing (not shown) disposed in cam follower hole 70 traverses cam groove 65 as cam 64 rotates, causing cam follower 68 to reciprocate from the proximal position of FIG. 36 to the distal position of FIG. 37. As a result, cam transfer 72, cannula transfer 74 and inner cannula 76 translate between their respective proximal positions of FIG. 36 and their respective distal positions of FIG. 37 when motor 62 and cam 64 rotate.

Motor 62 is preferably selected to have a rotational speed that allows inner cannula 76 to reciprocate from the position of FIG. 36 to the position of FIG. 37 and back to the position of FIG. 36 at a rate of at least about 1,000 reciprocations/minute. Reciprocation rates of at least about 1,200 reciprocations/minute are more preferred, and reciprocation rates of at least about 1,500 reciprocations/minute are even more preferred. Reciprocation rates of less than about 2,500 reciprocations/minute are preferred. Reciprocation rates of less than about 2,000 are more preferred, and reciprocation rates of less than about 1,800 reciprocations/minute are even more preferred. As best seen in FIG. 14, the rates of reciprocation of device 40 allow tissue to be severed into "snippets" 112 which are relatively smaller than "slug" tissue samples obtained by many prior devices. As the reciprocation continues, a continuum of severed tissue snippets 112 is obtained.

As mentioned previously, angled handpiece 342 allows a surgeon to grip device 340 with a single hand while keeping the hand out of a line of sight along inner cannula longitudinal axis L₁. In accordance with one exemplary method of use, device 340 is used to perform a tissue cutting procedure involving the cutting of a neurological target tissue. In one example, the target tissue is brain tissue, and in another example the target tissue is spinal tissue, for example, the tissue of an intervertebral disk. In certain exemplary methods, the tissue specimen being cut is a tumor or a lesion.

In accordance with the exemplary method, it is first determined whether the cutting operation will be a debulking operation or a fine shaving operation or a cutting operation that is somewhere in between a debulking and fine shaving operation. A given surgical procedure may also involve various combinations of these operations. A surgical access path is then created to the tissue sample of interest. In one embodiment, the surgical path is created and/or the target tissue is accessed using an "open" procedure in which the target tissue is open to the atmosphere (e.g., a full open craniotomy). In another embodiment, the surgical path is created and/or the target tissue is accessed using a "closed" procedure in which the target tissue is sealed from the atmosphere.

At this point, the distal end 79 of inner cannula 76 is located proximally of outer cannula opening 49 due to the use of an inner cannula stop position control of the type described previously. The maximum vacuum level to be applied to device 40 is then set using panel controls 138. Generally, higher vacuum levels will be used for debulking procedures than for fine shaving procedures as higher vacuum levels will tend to draw relatively larger sections of tissue into outer cannula opening 49. In one embodiment, the panel control 138 is a knob on console 134 that is rotated to set the desired maximum vacuum level.

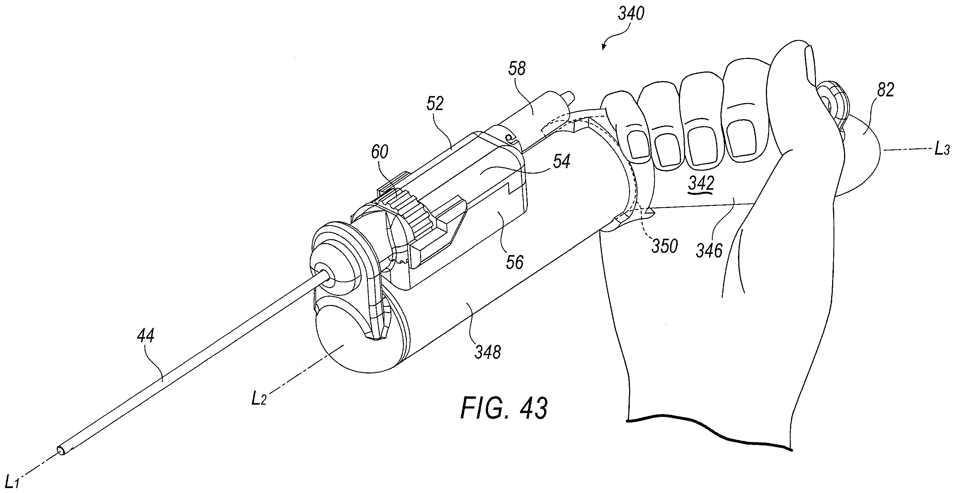

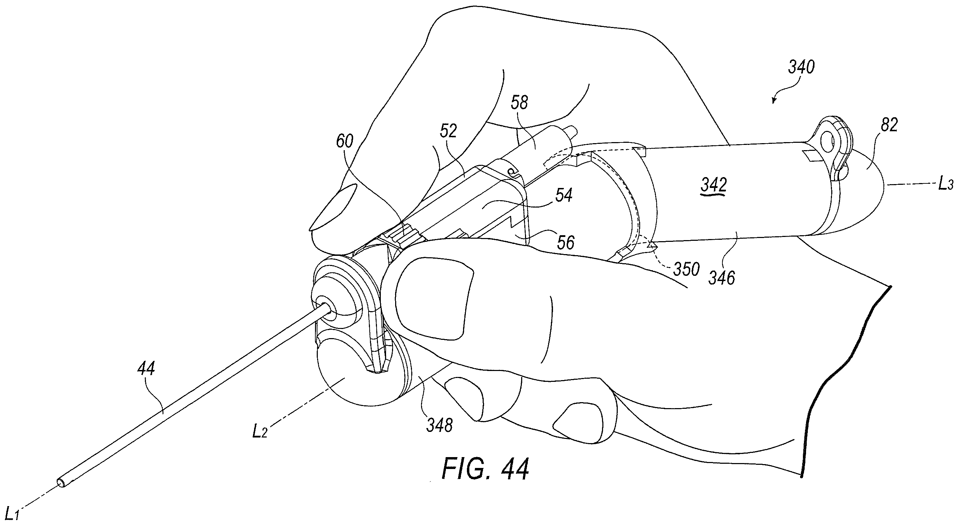

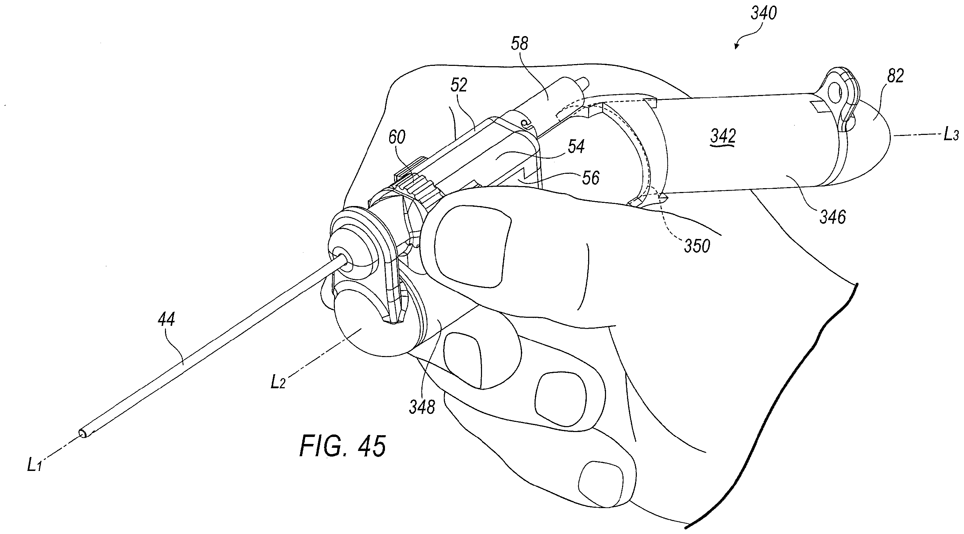

In one arrangement, device 340 is configured to be gripped with a single hand during a tissue cutting procedure. The surgeon may grip angled handpiece 342 in a number of ways. In one example, depicted in FIG. 43, the surgeon grips proximal handpiece section 346 in one hand with the thumb placed adjacent proximal-most housing portion 82 and the fourth finger placed adjacent distal handpiece section 348. Another grip is depicted in FIG. 44. In FIG. 44, distal housing section 348 is gripped with the fingers of one hand. As shown in the figure, the thumb is placed on one side of upper housing 52 and the index finger is placed on an upper surface of upper housing 52. Proximal handpiece section 346 rests between the base of the thumb and index finger. The index finger may be placed on rotation dial 60 to facilitate the rotation of outer cannula 44 and inner cannula 76. In yet another example, shown in FIG. 45, distal housing section 348 is again gripped with the fingers of one hand. However, the thumb is placed on one side of upper housing 52 and the index finger is placed on the other side of upper housing 52, with the proximal handpiece section resting between the base of the thumb and index finger.

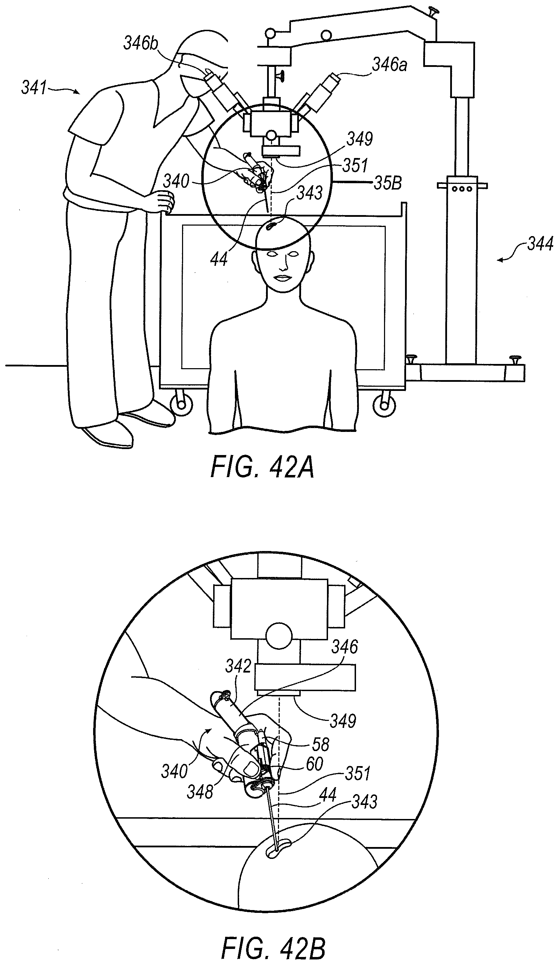

In one example, the surgeon may align outer cannula 44 with the target tissue by viewing the proximal portion of the upper housing 52 along a line of sight that is substantially collinear with inner cannula longitudinal axis L₁ and aligning axis L₁ with the target tissue. In certain cases, the handpiece angle α will preferably be large enough to prevent device 340 from bumping adjacent instruments. In one preferred embodiment depicted in FIG. 42A, tissue cutting device 340 is used to perform an open procedure using a microscope 344. In accordance with the embodiment, the lens 349 of the microscope 344 is aligned with the target tissue 343 so that the target tissue is in a line of sight 351 of the microscope's field of view. The outer cannula 44 is then positioned in the microscope's field of view between the lens 349 and the target tissue 343. The surgeon 341 views the tissue cutting device 340 and inserts outer cannula 44 to a location proximate the target tissue 343 and cuts the target tissue 343 in the manner described previously. In one example, the entire distal handpiece section 348 is placed in the microscope's field of view between the microscope lens 349 and the target tissue 343 along a direction parallel to line of sight 351. The surgeon 341 then inserts the outer cannula 44 to the desired location proximate the target tissue 343 to begin cutting. In one exemplary method, best seen in FIG. 42B, the surgeon 341 grasps the distal handpiece section 348 with the fingers of one hand while inserting the outer cannula 44 into the patient, and the proximal handpiece section 346 bends away from microscope 344. Thus, upper housing 52 and outer cannula 44 are positioned between distal handpiece section 348 and microscope line of sight 351. In addition, proximal handpiece portion 346 is not disposed between lens 349 and target tissue 343 in a direction along line of sight 351. Instead, proximal handpiece section 346 extends above the vertical location of lens 349. This configuration allows lens 349 to be placed more closely to target tissue 343 than would otherwise be possible if proximal handpiece section 346 were collinearly aligned with distal handpiece section 348. In the example depicted in FIG. 42B, tissue cutting device 340 is positioned such that a distance is defined between lens 349 and the proximal-most end of distal handpiece section 348, which is less than the length of proximal handpiece section 346 (i.e., the length from proximal-most housing section 82 to the vertex of the angle β that is defined between drive gear 310 and driven gear 312). Depending on the hand and the surgeon's orientation with respect to the target tissue 343, the surgeon may then rotate dial 60 to rotate outer cannula 44 about its own longitudinal axis as mentioned previously. Motor 62 may then be activated to reciprocate the inner cannula 76 as described previously (and as further illustrated in FIG. 40) to cut the target tissue, and the level of vacuum in inner cannula lumen 78 may be adjusted to achieve the desired degree of traction based on considerations such as whether a debulking or fine shaving operation is desired.

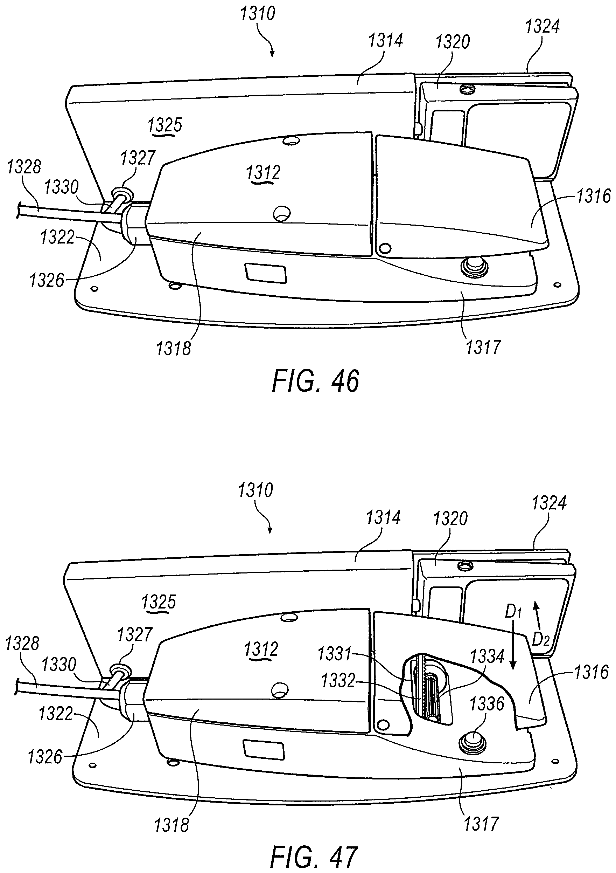

As discussed above, a foot pedal 144 may be used to activate motor 62 and/or to control the level of vacuum supplied to inner cannula lumen 78. In another exemplary embodiment, a foot actuator assembly is provided which includes multiple foot pedal assemblies for performing multiple operations. An exemplary embodiment of such a foot actuator assembly 1310 is depicted in FIG. 46. Foot actuator assembly 1310 includes multiple foot pedal assemblies, which in the embodiment of FIG. 46 includes foot pedal assembly 1312 and foot pedal assembly 1314. Foot actuator assembly 1310 is manipulable in a first direction D₁ to perform a first operation or set of operations and in a second direction D₂ to perform a second operation or set of operations. The directions D₁, and D₂ may be parallel and opposite one another or may define a variety of angles with respect to one another. In the embodiment of FIG. 46, D₁ is generally perpendicular to D₂, e.g., D₁ is vertically downward and D₂ is horizontal.

Foot pedal assembly 1312 comprises a support plate 1322 which preferably comprises a generally rigid material such as metal or hard plastic. Foot pedal assembly 1312 also comprises a distal housing section 1318, a proximal foot pedal 1316, and a proximal housing section 1317. Proximal foot pedal 1316 is operable in the direction D₁ to perform a first operation or set of operations with tissue removal device 40. Support plate 1322 provides a resistive force when pedal 1316 is depressed.

In a preferred embodiment, foot pedal assembly 1312 is operatively connected to proportional valve 1146, and therefore, operatively connected vacuum generator 1153 to variably adjust the vacuum level supplied to inner cannula lumen 78. Foot pedal assembly 1312 is also preferably operatively connected to motor 62 to cause it to rotate and thereby cause inner cannula 76 to reciprocate.

A cut-away view of foot pedal 1316 is provided in FIG. 47. As shown in the figure, foot pedal assembly 1312 comprises a switch 1336 and a potentiometer 1331. Switch 1336 is actuated by depressing pedal 1316 in direction D₁ and is used to activate motor 62. In the example of FIGS. 46-47, foot pedal 1316 is in an initial configuration wherein pedal 1316 is spaced apart from switch 1336 in the direction D₁. In a switching configuration (not separately shown), foot pedal 1316 contacts switch 1336 causing it to depress to activate motor 62. In certain examples, motor 62 is only activated if an inner cannula enable switch is also activated, as discussed further below.

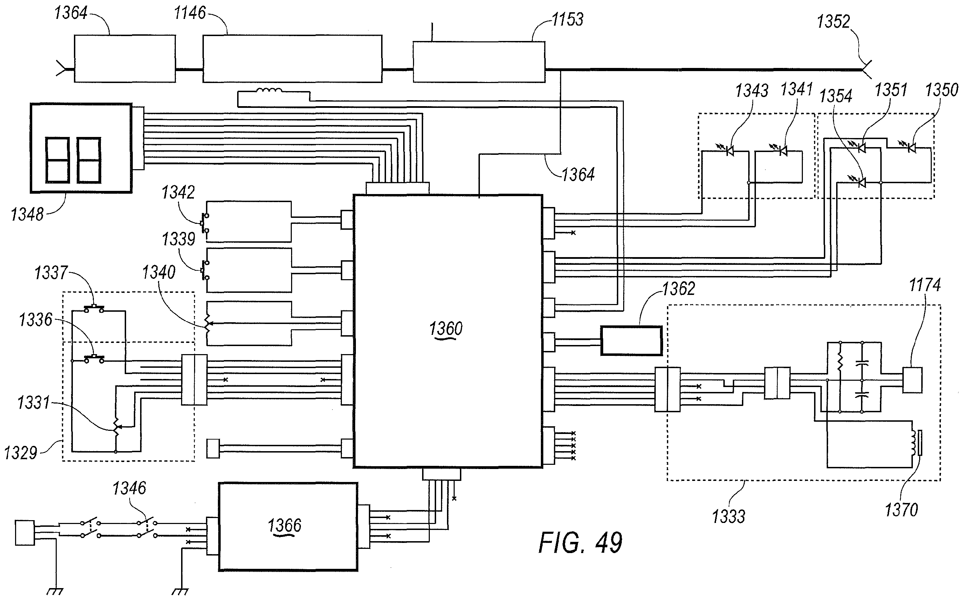

Foot pedal assembly 1314 comprises a support plate 1324 and a foot pedal 1320. One suitable commercially available foot pedal 1320 is the Linemaster^{®} Treadlite II, supplied by Linemaster Switch Corporation. Foot pedal assembly 1314 is preferably operatively connected to motor 62. In the example of FIG. 46, support plate 1324 is fixedly secured to support plate 1322 such as with mechanical fasteners, adhesives, welded connections, slot and tab connections, and/or combinations of the same. As a result, when a user depresses pedal 1320 in direction D₂, support plate 1324 provides a resistive force allowing pedal 1320 to abuttingly engage a switch (not separately shown in FIG. 46) that supplies power to motor 62. In one example, pedal 1320 activates an inner cannula (or cutter) enable switch 1337 (FIG. 49) that must be activated in order for power to be supplied to motor 62. In one exemplary embodiment, both foot pedal assembly 1312 and foot pedal assembly 1314 must be manipulated to activate motor 62 and initiate the reciprocation of inner cannula 76.

Support plate 1324 of foot pedal assembly 1314 is connected to a distal wiring shroud 1325. Distal wiring shroud 1325 and support plate 1324 define an enclosure with a hollow interior through which a wiring cable 1330 from foot pedal 1320 is routed. Cable 1330 is routed to grommet 1327 and into foot pedal assembly 1312 to electrically connect inner cannula enable switch 1337 to an input terminal in foot pedal assembly 1312. A conductor (e.g. a wire) is routed through cable 1330 and defines a signal path that electrically connects a corresponding output terminal in foot pedal assembly 1312 to a corresponding terminal on console board 1360. Similarly, inner cannula activation switch 1336 is electrically connected to a corresponding terminal on console board 1360 via a wire routed through output cable 1328. Console board 1360 may then be programmed to provide current to motor latching relay 1370 when both switch 1336 and 1337 are activated.

Potentiometer 1331 is used to open and close proportional valve 1146 to supply a vacuum generating gas, such as nitrogen, air, or another inert gas to vacuum generator 1153. In the embodiment of FIGS 46-47, potentiometer 1331 comprises a vertical gear 1332 that is attached at one end to foot pedal 1316 and which engages the teeth of a cylindrical gear 1334. Depressing or releasing foot pedal 1316 moves the vertical gear 1332 in the vertical direction, causing cylindrical gear 1334 to rotate. Potentiometer designs other than a vertical/cylindrical gear design may be used, including a flat, linear slide style of potentiometer. One suitable pedal assembly 1312 that may be used and which includes such a potentiometer is the Herga. 6253 Heavy Duty Foot Potentiometer supplied by Herga Electrical, Ltd.

Rotation of cylindrical gear 1334 provides a variable resistance, and therefore, a variable current, to proportional valve 1146. The current variation corresponds to a variation in the percentage of open flow area in proportional valve 1146, which thereby affects the flow of vacuum generating gas to vacuum generator 1153. As a result, potentiometer 1331 allows the user to variably adjust the vacuum level of inner cannula lumen 78 along a continuum from zero vacuum to the maximum vacuum level set with maximum aspiration controller 1340, as discussed further below. In the embodiment of FIG. 47, a separate switch 1336 is provided in foot pedal assembly 1312 to activate the inner cannula once it is enabled (by actuating pedal 1320 and activating inner cannula enable switch 1337). However, foot pedal assembly 1312 may also be configured so that once potentiometer 1331 is actuated (or once a threshold resistance is reached), the inner cannula 76 begins reciprocating; assuming again that the inner cannula enable switch 1337 has been activated, thus eliminating the need for switch 1336.

The foregoing configuration of foot actuator assembly 1310 advantageously allows a user to manipulate foot pedals 1320 and 1316 with a single foot. In one exemplary method, pedal 1316 can be depressed to a depressed configuration and the user can quickly pivot his or her foot to engage foot pedal 1320, preferably without lifting his or her foot off of pedal 1316 or without significantly changing the position of foot pedal 1316, and therefore, without significantly changing the inner cannula lumen vacuum level.

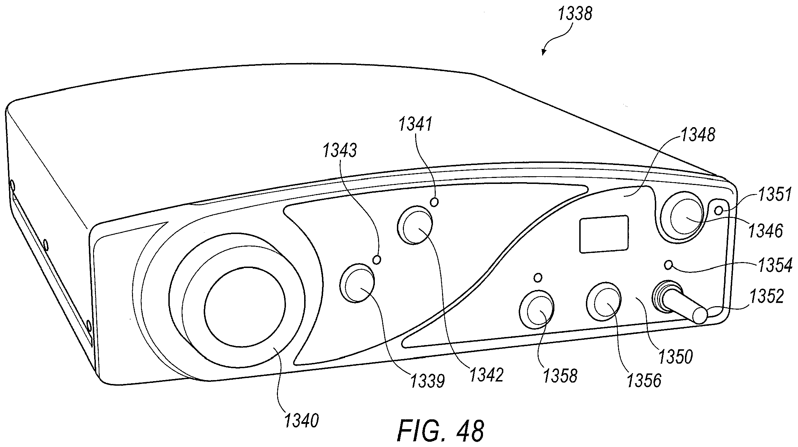

Like foot pedal 144, foot actuator assembly 1310 may also be used with an operator console such as operator console 1338, shown in FIG. 48. An exemplary circuit for tissue removal device 40, foot actuator assembly 1310 and console 1338 is depicted in FIG. 49. Power switch 1346 provides power to power supply 1366 from a source of AC current. Power supply 1366 acts as an AC to DC converter and provides direct current to console board 1360. Console 1338 comprises a maximum aspiration controller, which in the example of FIG. 48 is a rotatable dial potentiometer 1340. The user adjusts the rotational position of rotatable dial potentiometer 1340 to set the maximum vacuum level that may be supplied to inner cannula lumen 78 when foot pedal 1316 is fully actuated (e.g., fully depressed in the direction D₁ of FIG. 46). When rotatable dial 1340 is rotated to its maximum position (e.g., fully clockwise) and foot pedal 1316 is fully actuated (e.g., fully depressed), proportional valve 1146 is fully open to cause vacuum generator 1153 to provide the maximum available vacuum level to inner cannula lumen 78 via aspiration output line 1352 (FIG. 49).

In the embodiment of FIGS. 48 and 49, pressure regulator 1364, proportional valve 1146 and vacuum generator 1153 (e.g., a venturi device) are provided within console 1338. Although not shown in FIG. 48, console 1338 preferably includes a connector for connecting console 1338 to a source of vacuum generating gas (e.g., nitrogen). Pressure regulator 1364 may be a control valve that provides a regulated pressure to the inlet of proportional valve 1146 to better ensure that proportional valve 1146 produces consistent flow rates of vacuum generating gas at a given valve position or percentage open. Aspiration output connector 1352 is in fluid communication with vacuum generator 1153. The user may connect vacuum line 151c (FIG. 21A) to aspiration output connector 1352 and tissue collection canister 192 to fluidly couple vacuum generator 1153 to inner cannula lumen 78.

As indicated in FIG. 21A, instead of locating the vacuum generator 153, pressure regulator 1364, and proportional valve 1146 in a console, they may be located externally to a console. As shown in FIG. 49, vacuum measurement line 1364 is fluidly connected to aspiration output line 1352 and is connected to a vacuum sensor (not shown) located in console board 1360. The vacuum sensor provides an indication of the vacuum level in aspiration line 1352. LED display 1348 indicates the measured vacuum level to the user.

Cutter enable button 1342 duplicates the function of foot pedal 1320 and its switch 1337 (shown in FIG. 49), allowing the user to enable motor 62 to begin rotating once the surgeon begins to depress foot pedal 1316 sufficiently to activate switch 1336. Indicator 1341 alerts the surgeon that inner cannula 76 reciprocation has been enabled. In addition, sonic alert 1362 provides an audible indication (e.g., a beeping or steady tone) that inner cannula 76 has been enabled for reciprocation. As shown in FIG. 49, potentiometer 1331, inner cannula activation switch 1336, and inner cannula enable switch 1337 collectively define a foot switch sub assembly 1329.

Electrical output cable 1330 (FIG. 47) includes a conductor (e.g., a wire) that provides a signal path for transmitting a signal indicative of the position of inner cannula enable switch 1337 to console board 1360. The signal path is routed through foot pedal assembly 1312, hub 1326, and common output cable 1328. Electrical output signals from potentiometer 1331 and inner cannula activation switch 1336 are also transmitted to console board 1360 by conductors routed through common cable 1328. Common cable 1328 is connected to console footswitch connector 1356 to provide the necessary electrical connections between foot switch sub assembly 1329 and console board 1360.

Referring again to FIG. 49, inner cannula motor latching relay 1370 and inner cannula position sensor 1174 comprise a portion of a handpiece subassembly 1333. Thus, when both inner cannula enable switch 1337 and inner cannula activation switch 1336 are activated, current is supplied to inner cannula motor latching relay 1370 to cause motor 62 to rotate, as described previously. Console 1338 includes a handpiece connector 1358 for receiving a power cable that is connected to motor 62 via power cable port 84 (FIGS. 2-3).

Additional indicators may be provided on console 1338 to further indicate the status of tissue removal device 40, 340 and/or console 1338. For example, indicator 1351 (e.g., an LED) may be provided to indicate that power is being supplied to console 1338. Indicator 1354 may also be provided to indicate that aspiration is active (i.e., that potentiometer 1331 is outputting a valve opening signal to proportional valve 1146).

In certain embodiments, the aspiration pathway from outer cannula opening 49 to fluid collection canister 192 is primed before beginning a surgical procedure to prevent tissue occlusions from occurring due to the engagement of tissue samples with unlubricated surfaces. Accordingly, console 1338 includes a priming switch 1339 that briefly provides a pre-determined vacuum level to inner cannula lumen 78 when depressed by the user. In one exemplary method of use, the outer cannula 47 is inserted into a priming fluid (e.g., sterile saline) and the priming switch 1339 is depressed to aspirate the priming fluid through the outer cannula opening 49, through the inner cannula lumen 78 and into fluid collection canister 192. Console 1338 may also include a priming indicator 1343, such as an LED or other visible indicator, to indicate when priming is occurring.

Exemplary methods of using foot actuator assembly 1310 with console 1338 and tissue removal device 40 will now be described (use with tissue removal device 340 operates similarly). In accordance with one exemplary method, a source of a vacuum generating gas (e.g., nitrogen) is connected to console 1338 (connector not shown) so as to be in fluid communication with pressure regulator 1364. A power cable connected to motor 62 is routed through power cable port 84 in tissue removal device 40 (340) and is connected to handpiece connector 1358 on console 1338. Common outlet cable 1328 is connected (via a suitable cable connector) to footswitch connector 1356 on console 1338. Power button 1346 is pressed to turn on console 1338. Fluid container vacuum outlet line 151c (FIG. 21A) is connected to vacuum connector 1352, and vacuum inlet line 151b (FIG. 21A) is connected to proximally projecting portion 95 of seal holder 94 on the proximal end of tissue cutting device upper housing 52. Maximum aspiration controller 1340 is rotated to the desired position to set the maximum level of aspiration available when foot pedal 1316 is fully depressed in direction D₁.

A source of priming fluid, such as sterile water, is provided in a container and the outer cannula opening 49 is inserted to a distance below the level of the water. The priming switch 1339 is then pressed, causing the priming indicator 1343 to go on and thereby opening proportional valve 1146 and aspirating the priming fluid into outer cannula opening 49, through inner cannula lumen 78, and into fluid collection canister 192. This priming operation thusly lubricates the tissue sample aspiration path of tissue removal device 40, 340.

The target tissue is then accessed via an endoscopic or open approach, and outer cannula 47 is inserted proximate the target tissue. Outer cannula rotation dial 60 is rotated to adjust the circumferential orientation of outer cannula opening 49 as needed. The surgeon places one foot on foot pedal 1316 while leaving pedal 1316 in the fully undepressed position. If a tissue cutting operation is desired, the surgeon moves his or her foot to depress pedal 1320 in direction D₂ one time, thereby enabling inner cannula 76 and causing inner cannula enable indicator 1341 to activate. Alternatively, console inner cannula enable switch 1342 may be depressed instead of using pedal 1320. When the surgeon wishes to commence cutting, foot pedal 1316 is depressed in direction D₁ until switch 1336 is contacted and activated, at which point motor 62 will rotate to cause inner cannula 76 to reciprocate within outer cannula 47. The aspiration provided by vacuum generator 153 will draw tissue into outer cannula opening 49 where it will be severed by the distal end 79 of inner cannula 76. The severed tissue is then aspirated through inner cannula lumen 78 and into tissue collector 58. With the pedal 1316 at the desired position, the surgeon pivots his or her foot to again engage inner cannula enable switch 1320, thereby aspirating fluid and/or tissue without cutting any further tissue.

Foot actuator assembly 1310 is particularly well suited for operating tissue removal device in an aspiration wand mode followed by a tissue cutting mode. In accordance with one exemplary method, foot actuator assembly 1310 and tissue removal device 40 are connected to console 1338 as described above, as is tissue collection canister 192. The target tissue is again accessed using an endoscopic or open approach, and outer cannula 47 is inserted proximate the target tissue. After priming tissue removal device 40 (340) and setting the maximum aspiration level with controller 1340, the surgeon places one foot on foot pedal 1316 and depresses it in direction D₁ to obtain the desired degree of aspiration. If an inner cannula position control of the type described previously is provided, it may be used to adjust the position of inner cannula distal end 79 within outer cannula opening 49, and therefore, the amount of open area in outer cannula opening 49. At this point, inner cannula 76 is not reciprocating. By manipulating the position of outer cannula opening 49 and varying the position of foot pedal 1316, the surgeon may precisely determine the amount and nature of the tissue and/or fluids drawn into outer cannula opening 49. Once the tissue is drawn in and is ready to be cut, the surgeon pivots the foot that is on foot pedal 1316 in the direction D₂ to actuate foot pedal 1320, causing inner cannula 76 to reciprocate and sever the tissue drawn into outer cannula opening 49 while the vacuum level in inner cannula lumen 78 is at the level dictated by the position of foot pedal 1316. Accordingly, foot actuator assembly 1310 allows the user to manipulate the vacuum level at which cutting begins using a single foot to adjust foot pedals 1316 and 1320. Thus, manipulating foot actuator assembly 1310 in direction D₁ activates an aspiration operation and manipulating foot actuator assembly in direction D₂ activates a tissue cutting operation. Once tissue cutting is complete, foot pedal 1320 may again be engaged to discontinue tissue cutting while maintaining aspiration at a desired level.

It will be appreciated that the tissue cutting devices and exemplary methods described herein have broad applications. The foregoing embodiments were chosen and described in order to illustrate principles of the exemplary methods and apparatuses as well as some practical applications. The preceding description enables others skilled in the art to utilize the exemplary methods and apparatuses in various embodiments and with various modifications as are suited to the particular use contemplated. In accordance with the provisions of the patent statutes, the principles and modes of operation of this invention have been explained and illustrated in exemplary embodiments.

The scope of the present invention is defined by the following claims. However, it must be understood that this invention may be practiced otherwise than is specifically explained and illustrated without departing from its scope. It should be understood by those skilled in the art that various alternatives to the embodiments described herein may be employed in practicing the claims without departing from the scope as defined in the following claims. The scope of the invention should be determined, not with reference to the above description, but should instead be determined with reference to the appended claims, along with the full scope of equivalents to which such claims are entitled. It is anticipated and intended that future developments will occur in the arts discussed herein, and that the disclosed systems and exemplary methods will be incorporated into such future examples. Furthermore, all terms used in the claims are intended to be given their broadest reasonable constructions and their ordinary meanings as understood by those skilled in the art unless an explicit indication to the contrary is made herein. In particular, use of the singular articles such as "a," "the," "said," etc. should be read to recite one or more of the indicated elements unless a claim recites an explicit limitation to the contrary. It is intended that the following claims define the scope of the invention and that the apparatus within the scope of these claims and their equivalents be covered thereby. In sum, it should be understood that the invention is capable of modification and variation and is limited only by the following claims.

## Claims

1. A neurosurgical tissue removal assembly, comprising:
an endoscope (300) with an endoscope shaft (306);
a trocar (307) having a shaft (310) with a working channel (324) any an endoscope channel (326); and
a tissue removal device (40), wherein the tissue removal device comprises:
a handpiece (42) having a lower housing (50) disposed along a first longitudinal axis (L1) and an upper housing (52) disposed along a second longitudinal axis (L2) that is spaced apart from the first longitudinal axis,
an outer cannula (44) having an outer cannula lumen (110) an outer cannula proximal end (45), an outer cannula distal end (47), and an outer cannula opening (49) adjacent the outer cannula distal end, wherein the outer cannula is mounted in the upper housing along the second longitudinal axis for rotational movement and wherein the outer cannula opening defines a cutting edge (51) for severing tissue, and the outer cannula is removably insertable into the working channel,
an inner cannula (76) disposed in the outer cannula lumen and reciprocable within the outer cannula lumen, the inner cannula having an inner cannula lumen (78) an inner cannula proximal end (77), an inner cannula open distal end (79), a cutting edge at the inner cannula distal end (79), a living hinge (80), a cutting section (83), and a body section (81), with the hinge being located between the cutting section and the body section, wherein the cutting section is pivotable when the inner cannula reciprocates within the outer cannula lumen,
a motor (62) disposed in the lower housing portion, wherein the motor is operatively connected to the inner cannula so as to reciprocate the inner cannula within the outer cannula; and
a tissue collector (58) in fluid communication with the inner cannula lumen,
wherein at least a portion of the outer cannula is disposed in the working channel and at least a portion of the endoscope shaft is disposed in the endoscope channel; and
wherein rotation of the outer cannula about the second longitudinal axis with respect to the handpiece causes the inner cannula to rotate about the second.

2. The neurological tissue removal assembly of claim 1, wherein the tissue cutting device handpiece is spaced apart from the trocar and wherein an endoscope eyepiece (302) is located between the trocar and the handpiece in a direction along the length of the endoscope.

3. The neurological tissue removal assembly of claim 1, wherein the motor has a rotating shaft (66) that is substantially parallel to and spaced apart from the inner cannula.

4. The tissue removal device of claim 1, wherein the housing (50) has a proximal end (46), a distal end (48), and a midpoint (MP) along the housing longitudinal axis, the device has a center of gravity, and the center of gravity is located between the midpoint of the housing and the distal end of the housing along the direction of the housing longitudinal axis.

5. The tissue removal device of claim 4, further comprising a drive assembly (63) disposed in the lower housing, wherein the drive assembly is located between the midpoint of the housing and the distal end of the housing.

6. The tissue removal device of claim 5, wherein the drive assembly comprises a cam (64), and the rotation of the motor causes the cam to rotate.

7. The tissue removal device of claim 6, wherein the cam (64) has a length, a surface, a continuous channel (65) defined in the surface, two cam channel apexes (65a, 65b) that are spaced apart from one another along the length of the cam, and a cam longitudinal axis, wherein the cam longitudinal axis is substantially parallel to and spaced apart from the second longitudinal axis.

8. The tissue removal device of claim 7, further comprising a cam follower (68) connected to the inner cannula, wherein the cam follower is disposed in the channel and moves along the channel when the cam rotates about the cam longitudinal axis.

9. The tissue removal device of claim 1, wherein the inner cannula has a radial direction, and when the inner cannula and the outer cannula are in the fixed circumferential orientation, the hinge is spaced apart from the outer cannula opening in the radial direction.

10. The tissue removal device of claim 1, further comprising an inner sleeve (88) disposed about the outer cannula and seated within the upper house, and a rotation wheel (60) connected to the inner sleeve and the inner cannula, such that rotation of the rotation wheel causes the outer cannula to rotate about the first longitudinal axis.

11. The tissue removal device of claim 10, wherein the rotation wheel is partially disposed in the upper housing such that a user engagement surface of the rotation wheel projects away from the upper housing.

12. The tissue removal device of claim 6, further comprising a cam transfer (74), wherein the cam transfer has a first end (72a) that engages the cam and a second end (72b) that engages the inner cannula, such that when the cam rotates, the cam transfer reciprocates in a direction that is substantially parallel to the second longitudinal axis.

13. The tissue removal device of claim 6, wherein the motor is connected to a drive gear (310), the cam is connected to a driven gear (312) and the drive gear engages the driven gear.

14. The tissue removal system of claim 1, further comprising a foot actuator assembly (1310) operatively connected to the tissue removal device, wherein the foot actuator assembly is manipulable in a first direction to perform a first operation and a second direction to perform a second operation.

15. The tissue removal system of claim 14, wherein the foot actuator assembly comprises a first foot pedal (1316) that is manipulable in the first direction and a second foot pedal (1320) that is manipulable in the second direction, wherein the first foot pedal is operatively connected to a vacuum generator (1153) that is in fluid communication with the inner cannula lumen.

16. The tissue removal system of claim 15, wherein the first foot pedal and the second foot pedal are operatively connected to the motor such that the first foot pedal and the second foot pedal must be manipulated to operate the motor.

17. The tissue removal system of claim 14, wherein when the foot actuator assembly is manipulated in the first direction, a vacuum level is generated in the inner cannula lumen,
wherein when the foot actuator assembly is manipulated in the second direction, reciprocation of the inner cannula is enabled, and wherein when the foot actuator assembly is manipulated in the first direction and the second direction simultaneously, the inner cannula reciprocates within the outer cannula lumen.

18. The tissue removal system of claim 14, wherein the foot actuator assembly is manipulable in the first direction to adjust an inner cannula vacuum level along a continuum of vacuum levels that are no greater than a preselected maximum vacuum level, such that the foot actuator assembly is manipulable from a first position to a second position along the first direction, and when the foot actuator assembly is in the second position, the inner cannula vacuum level equals the preselected maximum vacuum level.

19. The tissue removal system of claim 1, further comprising an inner cannula stop position control system, wherein the inner cannula stop position control system comprises an inner cannula position sensor (174) and a motor control unit (160).

## Patentansprüche

1. Neurochirurgische Gewebeentfernungseinheit, umfassend:
ein Endoskop (300) mit einer Endoskopwelle (306);
einen Trokar (307) mit einer Welle (310) mit einem Arbeitskanal (324) und
einem Endoskopkanal (326); und eine Gewebeentfernungsvorrichtung (40),
wobei die Gewebeentfernungsvorrichtung umfasst:
ein Handstück (42) mit einem unteren Gehäuse (50), angeordnet entlang einer ersten Längsachse (L1), und einem oberen Gehäuse (52), angeordnet einer zweiten Längsachse (L2), die von der ersten Längsachse beabstandet ist,
eine äußere Kanüle (44) mit einem äußeren Kanülenlumen (110), einem proximalen Ende der äußeren Kanüle (45), einem distalen Ende der äußeren Kanüle (47), und einer Öffnung der äußeren Kanüle (49) anliegend am distalen Ende der äußeren Kanüle, wobei die äußere Kanüle im oberen Gehäuse entlang der zweiten Längsachse für eine Drehbewegung montiert ist, und wobei die Öffnung der äußeren Kanüle einen Schneiderand (51) zum Trennen von Gewebe definiert, und die äußere Kanüle abnehmbar in den Arbeitskanal eingeführt werden kann,
eine innere Kanüle (76), angeordnet im äußeren Kanülenlumen und hin- und herbewegbar im äußeren Kanülenlumen, wobei die innere Kanüle ein inneres Kanülenlumen (78), ein proximales Ende der inneren Kanüle (77), ein offenes distales Ende der inneren Kanüle (79), einen Schneiderand am distalen Ende der inneren Kanüle (79), ein Filmscharnier (80), einen Schneideabschnitt (83) und einen Körperabschnitt (81) hat, wobei sich das Scharnier zwischen dem Schneideabschnitt und dem Körperabschnitt befindet, wobei der Schneideabschnitt schwenkbar ist, wenn sich die innere Kanüle in dem äußeren Kanülenlumen hin- und herbewegt, ein Motor (62) im unteren Gehäuseabschnitt angeordnet ist, wobei der Motor operativ mit der inneren Kanüle verbunden ist, um die innere Kanüle in der äußeren Kanüle hin- und herzubewegen; und
einen Gewebekollektor (58) in Fluidverbindung mit dem inneren Kanülenlumen, wobei mindestens ein Abschnitt der äußeren Kanüle im Arbeitskanal angeordnet ist und mindestens ein Abschnitt der Endoskopwelle im Endoskopkanal angeordnet ist; und
wobei eine Drehung der äußeren Kanüle um die zweite Längsachse in Bezug auf das Handstück die innere Kanüle veranlasst, um die zweite zu drehen.

2. Neurologische Gewebeentfernungseinheit gemäß Anspruch 1, wobei das Handstück der Gewebeschneidevorrichtung vom Trokar beabstandet ist und wobei ein Endoskopokular (302) zwischen dem Trokar und dem Handstück in einer Richtung entlang der Länge des Endoskops angeordnet ist.

3. Neurologische Gewebeentfernungseinheit gemäß Anspruch 1, wobei der Motor eine drehende Welle (66) hat, die im Wesentlichen parallel zu und beabstandet von der inneren Kanüle ist.

4. Gewebeentfernungsvorrichtung gemäß Anspruch 1, wobei das Gehäuse (50) ein proximales Ende (46), ein distales Ende (48) und einen Mittelpunkt (MP) entlang der Gehäuselängsachse hat, die Vorrichtung einen Schwerpunkt hat, und sich der Schwerpunkt zwischen dem Mittelpunkt des Gehäuses und dem distalen Ende des Gehäuses entlang der Richtung der Gehäuselängsachse befindet.

5. Gewebeentfernungsvorrichtung gemäß Anspruch 4, ferner umfassend eine Antriebseinheit (63), angeordnet im unteren Gehäuse, wobei sich die Antriebseinheit zwischen dem Mittelpunkt des Gehäuses und dem distalen Ende des Gehäuses befindet.

6. Gewebeentfernungsvorrichtung gemäß Anspruch 5, wobei die Antriebseinheit eine Nocke (64) umfasst, und die Drehung des Motors ein Drehen der Nocke veranlasst.

7. Gewebeentfernungsvorrichtung gemäß Anspruch 6, wobei die Nocke (64) eine Länge, eine Oberfläche, einen durchgehenden Kanal (65) definiert in der Oberfläche, zwei Nockenkanalspitzen (65a), 65b), die voneinander entlang der Länge der Nocke beabstandet sind, und eine Nockenlängsachse hat, wobei die Nockenlängsachse im Wesentlichen parallel zu und beabstandet von der zweiten Längsachse ist.

8. Gewebeentfernungsvorrichtung gemäß Anspruch 7, ferner umfassend einen Nockenstößel (68) verbunden mit der inneren Kanüle, wobei der Nockenstößel im Kanal angeordnet ist und sich entlang des Kanals bewegt, wenn die Nocke um die Nockenlängsachse dreht.

9. Gewebeentfernungsvorrichtung gemäß Anspruch 1, wobei die innere Kanüle eine radiale Richtung hat, und wenn die innere Kanüle und die äußere Kanüle in der festgelegten Umfangsausrichtung ist, ist das Scharnier von der Öffnung der äußeren Kanüle in der radialen Richtung beabstandet.

10. Gewebeentfernungsvorrichtung gemäß Anspruch 1, ferner umfassend eine innere Hülse (88) angeordnet um die äußere Kanüle und sitzend im oberen Gehäuse, und ein Drehrad (60) verbunden mit der inneren Hülse und der inneren Kanüle, sodass eine Drehung des Drehrads die äußere Kanüle veranlasst, um die erste Längsachse zu drehen.

11. Gewebeentfernungsvorrichtung gemäß Anspruch 10, wobei das Drehrad teilweise im oberen Gehäuse angeordnet ist, sodass eine Benutzereingriffsfläche des Drehrads weg vom oberen Gehäuse zeigt.

12. Gewebeentfernungsvorrichtung gemäß Anspruch 6, ferner umfassend eine Nockenübergang (74), wobei der Nockenübergang ein erstes Ende (72a) hat, das die Nocke eingreift, und ein zweites Ende (72b), das die innere Kanüle eingreift, sodass wenn die Nocke dreht, der Nockenübergang sich in einer Richtung hin- und herbewegt, die im Wesentlichen parallel zur zweiten Längsachse ist.

13. Gewebeentfernungsvorrichtung gemäß Anspruch 6, wobei der Motor mit einem Antriebszahnrad (310) verbunden ist, die Nocke mit einem angetriebenen Zahnrad (312) verbunden ist, und das Antriebszahnrad das angetriebene Zahnrad eingreift.

14. Gewebeentfernungssystem gemäß Anspruch 1, ferner umfassend eine Fußbetätigungseinheit (1310), die operativ mit der Gewebeentfernungsvorrichtung verbunden ist, wobei die Fußbetätigungseinheit in eine erste Richtung manipuliert werden kann, um einen ersten Vorgang auszuführen, und in eine zweite Richtung, um einen zweiten Vorgang auszuführen.

15. Gewebeentfernungssystem gemäß Anspruch 14, wobei die Fußbetätigungseinheit ein erstes Fußpedal (1316) umfasst, das in die erste Richtung manipuliert werden kann, und ein zweites Fußpedal (1320), das in die zweite Richtung manipuliert werden kann, wobei das erste Fußpedal operativ mit einem Vakuumgenerator (1153) verbunden ist, der in Fluidverbindung mit dem inneren Kanülenlumen ist.

16. Gewebeentfernungssystem gemäß Anspruch 15, wobei das erste Fußpedal und das zweite Fußpedal operativ mit dem Motor verbunden sind, sodass das erste Fußpedal und das zweite Fußpedal manipuliert werden müssen, um den Motor zu betreiben.

17. Gewebeentfernungssystem gemäß Anspruch 14, wobei wenn die Fußbetätigungseinheit in die erste Richtung manipuliert wird, ein Vakuumpegel im inneren Kanülenlumen erzeugt wird, wobei wenn die Fußbetätigungseinheit in die zweite Richtung manipuliert wird, eine Hin- und Herbewegung der inneren Kanüle ermöglicht wird, und wobei wenn die Fußbetätigungseinheit gleichzeitig in die erste Richtung und in die zweite Richtung manipuliert wird, sich die innere Kanüle im äußeren Kanülenlumen hin- und herbewegt.

18. Gewebeentfernungssystem gemäß Anspruch 14, wobei die Fußbetätigungseinheit in die erste Richtung manipuliert werden kann, um einen Vakuumpegel der inneren Kanüle entlang einem Kontinuum von Vakuumpegeln einzustellen, die nicht größer als ein vorgewählter maximaler Vakuumpegel sind, sodass die Fußbetätigungseinheit von einer ersten Position zu einer zweiten Position entlang der ersten Richtung manipuliert werden kann, und wenn die Fußbetätigungseinheit in der zweiten Position ist, entspricht der Vakuumpegel der inneren Kanüle dem vorgewählten maximalen Vakuumpegel.

19. Gewebeentfernungssystem gemäß Anspruch 1, ferner umfassend ein Steuersystem für eine Stoppposition der inneren Kanüle, wobei das Steuersystem für eine Stoppposition der inneren Kanüle einen Positionssensor der inneren Kanüle (174) und eine Motorsteuereinheit (160) umfasst.

## Revendications

1. L'ensemble d'élimination de tissu neurochirurgical, comprend :
un endoscope (300) avec un arbre d'endoscope (306) ;
un trocart (307) ayant un arbre (310) avec un canal de travail (324) et un canal d'endoscope (326), et un dispositif d'enlèvement de tissu (40) dans lequel le dispositif d'enlèvement de tissu comprend :
une pièce à main (42) comportant un boîtier inférieur (50) disposé le long d'un premier axe longitudinal (L1) et un boîtier supérieur (52) disposé le long d'un deuxième axe longitudinal (L2) espacé du premier axe longitudinal,
une canule externe (44) ayant une lumière de canule externe (110), une extrémité proximale de canule externe (45), une extrémité distale de canule externe (47) et une ouverture de canule externe (49) adjacente à l'extrémité distale de canule externe, la canule externe est montée dans le boîtier supérieur le long du deuxième axe longitudinal pour un mouvement de rotation et dans lequel l'ouverture de canule externe définit un bord de coupe (51) pour découper le tissu et la canule externe peut être insérée de manière amovible dans le canal de travail,
une canule interne (76)
disposée dans la lumière de canule externe et pouvant se déplacer en va-et-vient à l'intérieur de la lumière de canule externe, la canule interne ayant une lumière de canule interne (78) ; une extrémité proximale de canule interne (77), une extrémité distale ouverte de canule interne (79), un bord de coupe à l'extrémité distale de canule interne (79), une charnière vivante (80), une section de coupe (83) et une section de corps (81), la charnière étant située entre la section de coupe et la section de corps, dans laquelle la section de coupe peut pivoter lorsque la canule interne se déplace en va-et-vient à l'intérieur de la lumière de canule externe,
un moteur (62) disposé dans la partie de boîtier inférieure, dans lequel le moteur est connecté de manière fonctionnelle à la canule interne de manière à faire alterner la canule interne à l'intérieur de la canule externe ; et
un collecteur de tissus (58) en communication fluidique avec la lumière interne de la canule,
dans lequel au moins une partie de la canule externe est disposée dans le canal de travail et au moins une partie de l'arbre d'endoscope est disposée dans le canal d'endoscope ; et
dans lequel la rotation de la canule externe autour du second axe longitudinal par rapport à la pièce à main amène la canule interne à tourner autour de la seconde.

2. L'ensemble d'enlèvement de tissu neurologique selon la revendication 1, dans
lequel la pièce à main de dispositif de coupe de tissu est espacée du trocart et dans lequel un oculaire d'endoscope (302) est situé entre le trocart et la pièce à main dans une direction le long de la longueur de l'endoscope.

3. L'ensemble d'enlèvement de tissu neurologique selon la revendication 1, dans lequel le moteur comporte un arbre rotatif (66) qui est sensiblement parallèle à et écarté de la canule interne.

4. Le dispositif d'enlèvement de tissu selon la revendication 1, dans lequel le boîtier (50) présente une extrémité proximale (46), une extrémité distale (48) et un point médian (MP)
le long de l'axe longitudinal du boîtier, le dispositif présente un centre de gravité,
et le centre de gravité est situé entre le point médian du boîtier et l'extrémité distale du boîtier suivant la direction de l'axe longitudinal du boîtier.

5. Le dispositif d'enlèvement de tissu selon la revendication 4, comprend en outre un ensemble d'entraînement (63) disposé dans le boîtier inférieur, dans lequel l'ensemble d'entraînement est situé entre le point médian du boîtier et l'extrémité distale du boîtier.

6. Le dispositif d'enlèvement de tissu selon la revendication 5, dans lequel l'ensemble d'entraînement comprend une came (64), et la rotation du moteur fait tourner la came.

7. Le dispositif d'enlèvement de tissu selon la revendication 6, dans lequel la came (64) a une longueur, une surface, un canal continu (65) défini dans la surface, deux sommets de canal de came (65a, 65b) espacés l'un de l'autre le long de la longueur de la came et un axe longitudinal de came, dans lequel l'axe longitudinal de la came est sensiblement parallèle et espacé du deuxième axe longitudinal.

8. Le dispositif d'enlèvement de tissu selon la revendication 7, comprend en outre un suiveur de came (68) relié à la canule interne, dans lequel le suiveur de came est disposé dans le canal et se déplace le long du canal lorsque la came tourne autour de l'axe longitudinal de came.

9. Le dispositif d'élimination de tissu selon la revendication 1, dans lequel la canule interne a une direction radiale et lorsque la canule interne et la canule externe sont dans l'orientation circonférentielle fixe, la charnière est espacée de la canule externe s'ouvrant dans la direction radiale.

10. Le dispositif d'enlèvement de tissu selon la revendication 1, comprend en outre un manchon intérieur (88) disposé autour de la canule externe et assis à l'intérieur de la chambre haute, et une roue de rotation (60) reliée au manchon intérieur et à la canule interne, de la roue de rotation provoque la rotation de la copule externe autour du premier axe longitudinal.

11. Le dispositif d'enlèvement de tissu selon la revendication 10, dans lequel la roue de rotation est partiellement disposée dans le logement supérieur de sorte qu'une surface d'engagement de l'utilisateur de la roue de rotation s'éloigne du logement supérieur.

12. Le dispositif d'enlèvement de tissu selon la revendication 6, comprend en outre un transfert de came (74), dans lequel le transfert de came comporte une première extrémité (72a) qui s'engage dans la came et une seconde extrémité (72b) qui engage la canule interne, de sorte que lorsque la came tourne, le transfert de came se déplace en va-et-vient dans une direction qui est sensiblement parallèle au second axe longitudinal.

13. Le dispositif d'enlèvement de tissu selon la revendication 6, dans lequel le moteur est relié à un engrenage d'entraînement (310), la came est reliée à un engrenage entraîné (312) et l'engrenage d'entraînement engage le pignon mené.

14. Le système d'élimination de tissu selon la revendication 1, comprend en outre un ensemble actionneur de pied (1310) relié fonctionnellement au dispositif d'enlèvement de tissu, dans lequel l'ensemble actionneur de pied peut être manipulé dans une première direction pour effectuer une première opération et une seconde direction pour exécuter une deuxième opération.

15. Le système d'élimination de tissu ou revendication 14, dans lequel l'ensemble actionneur de pied comprend une première pédale (1316) manipulable dans la première direction et une seconde pédale (1320) manipulable dans la seconde direction, dans laquelle le premier pied pédale est reliée fonctionnellement à un générateur de vide (1153) qui est en communication de fluide avec la lumière interne de la canule.

16. Le système d'élimination de tissu selon la revendication 15, dans lequel la première pédale et la seconde pédale sont reliées fonctionnellement au moteur de sorte que la première pédale et la seconde pédale doivent être manipulées pour faire fonctionner le moteur

17. Le système d'élimination de tissu selon la revendication 14, dans lequel, lorsque l'ensemble actionneur de pied est manipulé dans la première direction, un niveau de vide est généré dans la lumière interne de la canule,
dans lequel, lorsque l'ensemble actionneur de pied est manipulé dans la seconde direction,
le mouvement alternatif de la canule interne est activé, dans lequel, lorsque l'ensemble d'actionneur de pied est manipulé dans la première direction et la seconde direction simultanément, la canule interne se déplace en va-et-vient à l'intérieur de la lumière de canule externe.

18. Le système d'élimination de tissu selon la revendication 14, dans lequel l'ensemble d'actionneur de pied peut être manipulé dans la première direction pour ajuster un niveau de vide de canule interne le long d'un continuum de niveaux de vide qui n'est pas supérieur à un niveau de vide maximal présélectionné, de telle sorte que l'ensemble actionneur de pied peut être manipulé d'une première position à une seconde position le long de la première direction et lorsque l'ensemble actionneur de pied est dans la seconde position, le niveau de vide de canule interne est égal au niveau de vide maximal présélectionné.

19. Le système d'enlèvement de tissu selon la revendication 1, comprend en outre un système de commande de position d'arrêt de canule interne, dans lequel le système de commande de position d'arrêt de canule interne comprend un capteur de position de canule interne (174) et une unité de commande de moteur (160).
